(19) Europäisches Patentamt
European Patent Office
Office européen des brevets

(11) EP 1 541 560 A1

(12) EUROPEAN PATENT APPLICATION
published in accordance with Art. 158(3) EPC

(43) Date of publication:
**15.06.2005 Bulletin 2005/24**

(21) Application number: **03766703.7**

(22) Date of filing: **01.08.2003**

(51) Int Cl.[7]: **C07D 221/16**, C07D 401/12, C07D 405/12, A61K 31/473, A61P 5/24, A61P 7/00, A61P 7/06, A61P 15/00, A61P 15/10, A61P 19/10, A61P 35/00, A61P 43/00

(86) International application number:
**PCT/JP2003/009815**

(87) International publication number:
**WO 2004/013104 (12.02.2004 Gazette 2004/07)**

(84) Designated Contracting States:
**AT BE BG CH CY CZ DE DK EE ES FI FR GB GR HU IE IT LI LU MC NL PT RO SE SI SK TR**
Designated Extension States:
**AL LT LV MK**

(30) Priority: **01.08.2002 JP 2002225300**

(71) Applicant: **Kaken Pharmaceutical Co., Ltd.**
**Tokyo 113-8650 (JP)**

(72) Inventors:
- **MIYAKAWA, M, Central Res Lab, Kaken Pharma Co, Ltd Kyoto-shi, Kyoto 607-8042 (JP)**
- **OGURO, N., Central Res. Lab., Kaken Parma. Co, Ltd Kyoto-shi, Kyoto 607-8042 (JP)**
- **HANADA, K., Central Res. Lab, Kaken Pharma Co, Ltd Kyoto-shi, Kyoto 607-8042 (JP)**
- **FURUYA, K., Central Res. Lab, Kaken Pharma Co, Ltd Kyoto-shi, Kyoto 607-8042 (JP)**
- **YAMAMOTO, N, Central Res Lab, Kaken Pharma Co, Ltd Kyoto-shi, Kyoto 607-8042 (JP)**

(74) Representative: **HOFFMANN EITLE**
**Arabellastrasse 4**
**81925 München (DE)**

# (54) NOVEL TETRAHYDROQUINOLINE DERIVATIVES

(57) The present invention provides tetrahydroquinoline derivatives of the following general formula (I) or salts thereof

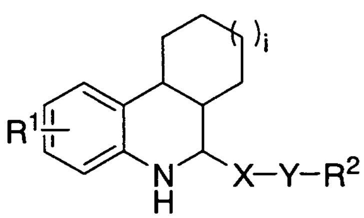

(I)

where $R^1$, $R^2$, X, Y and i are as defined in claim 1, which do not exhibit excessive action on the prostate, but which exhibit strong androgen receptor agonistic action particularly on skeletal muscle tissue and bone tissue, and pharmaceuticals comprising such derivatives or their salts as active ingredients.

EP 1 541 560 A1

## Description

### TECHNICAL FIELD

This invention relates to tetrahydroquinoline derivatives or salts thereof which do not exhibit excessive action on the prostate, but which exhibit particularly strong androgen receptor agonism on bone tissue and skeletal muscle tissue, and pharmaceuticals containing such derivatives or their salts.

### BACKGROUND ART

Androgens are a generic name for C19 steroids. They are sex hormones important for the normal sexual differentiation and growth of males, masculinization at puberty, activation of initial spermatogenesis in the testes, and maintenance of male function. About 90% of androgens are produced by Leydig cells of the testes, the remaining 10% by the adrenal gland, mainly as testosterone, and secreted into the blood. Testosterone is taken up into target cells, and converted by 5α-reductase into dihydrotestosterone (DHT) with potent biological activity. DHT, as well as testosterone, plays an important role in the development of male secondary sex characteristics (growth of sebaceous glands, acne, development of body hair, voice deepening, development of beards), growth of external genitalia (penis, testis), growth of accessory genital organs (prostate, seminal vesicles), sexual impulse, and occurrence of erection.

Aside from these major actions, androgens have actions other than those on the reproductive system, such as anabolic action (e.g., increases in skeletal muscles and bone mass, and erythropoiesis promoting action), and suppression of gonadotropin secretion. Target cells for androgens are not only localized in external and accessory genital tissues, but are also widely distributed in the brain, pituitary gland, muscular tissues, bones, and kidneys (N. Engl. J. Med. 334, 707-714, 1996).

In addition to these roles, androgens are reported to show an anti-inflammatory action. Nowadays, it is becoming clear that androgens attenuate arthritis and autoimmune disease by inhibiting the proliferation of inflammatory cells or suppressing the production of cytokines such as IL-6 (Ann. Rheum. Dis. 55, 811-815, 1996).

All androgenic actions are mediated through androgen receptor (hereinafter referred to as AR) having a molecular weight of about 100,000 which is present in the nuclei of target cells. The gene of AR was cloned by Chang et al. and Lubahn et al. in 1988. Their studies demonstrated that AR has a similar structure to estrogen, progesterone, mineral corticoid and glucocorticoid receptors and they build a family of nuclear steroid receptors (Science 240, 324-326, 327-330, 1988). Androgens with high liposolubility penetrate the target cell membrane by passive diffusion, and bind to the hormone-binding region of AR specifically and with high affinity to form dimers, which bind to an androgen responsive DNA region (androgen response element: ARE) localized upstream from a particular gene. As a result, transcription of the target gene is initiated to induce the expression of mRNA, thereby producing a functional protein responsible for an androgenic action, thus exhibiting this action (Trend in Endocrinology and Metabolism 9, 317-324, 1998). In this mechanism, compounds which bind to AR and show similar actions to those of natural ligands such as testosterone are defined as agonists.

As the AR agonists, androgenic steroid preparations such as testosterone esters and other derivatives are currently used in the treatment of osteoporosis, wasting diseases, male hypogonadism, etc.

However, these androgenic steroid preparations can cause side effects inherent in steroid preparations, such as hepatic dysfunction and gastrointestinal disorder, and may develop androgen-dependent tumor (e.g. prostatic cancer) or prostatic hypertrophy, or aggravate symptoms of these diseases, because they act excessively on the prostate if administered to male patients, especially to elderly patients. If these preparations are administered to female patients, they pose major problems of virilizing actions, such as changes in the vocal cord (male-like hoarseness), hypertrichosis of the body trunk, alopecia and acne.

Hence, nonsteroidal AR agonists which do not show excessive action on the prostate and are minimal in side effects are desired for the treatment of hypogonadism, and have been under research and development. However, globally recognized compounds are yet to be created.

In the case of drugs indicated for osteoporosis and wasting disease, compounds which exhibit potent AR agonism particularly on bone tissue and skeletal muscle tissue are desired, but no such compounds have been created yet.

### DISCLOSURE OF THE INVENTION

The present invention has been accomplished in view of the treatment of such AR mediated diseases and researches on their treatment. An object of the present invention is to provide novel nonsteroidal compounds or salts thereof which do not exhibit excessive action on the prostate as is observed with androgen steroid preparations, but which exhibit particularly strong AR agonism on bone tissue and skeletal muscle tissue. Another object of the present

invention is to provide pharmaceuticals comprising such compounds or salts thereof as an active ingredient.

The inventors of the present invention diligently conducted studies in an attempt to attain the above objects. As a result, they have found that among tetrahydroquinoline derivatives, specified compounds of the following formula (I) (hereinafter referred to as "compounds of the present invention") do not act excessively on the prostate, but exhibit particularly strong AR agonism on bone tissue and skeletal muscle tissue. Based on these findings, they have accomplished this invention.

That is, according to one embodiment, the present invention relates to a tetrahydroquinoline derivative represented by the following formula (I), or pharmacologically acceptable salts thereof:

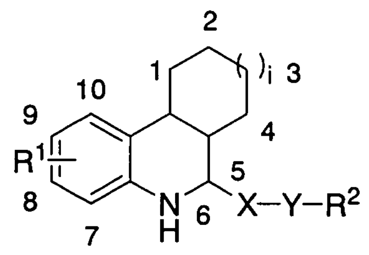

(I)

(numbers 1 - 10 used in the above formula (I) assume the case where i is 1 and, if i is 0, the position of number 3 is absent, so that numbers 4 - 10 are replaced by numbers 3 - 9 to indicate the respective positions for the purpose of the following explanation)

where $R^1$ represents a nitro group or a cyano group; i represents 0 or 1;

X represents an alkylene group having 1 - 5 carbon atoms which may be substituted by a substituent selected from the group consisting of an alkyl group having 1 - 6 carbon atoms and a cycloalkyl group having 3 - 7 carbon atoms;

Y represents $-NR^3CO-$, $-NR^3SO_2-$, $-NR^3CONH-$ or $-NR^3CSNH-$ (where $R^3$ represents a hydrogen atom, an alkyl group having 1 - 6 carbon atoms, a cycloalkyl group having 3 - 7 carbon atoms, or an aralkyl group having 7 - 9 carbon atoms); and

$R^2$ represents a phenyl group which may be substituted by 1 - 3 independent $R^4$'s, or a heteroaryl group which may be substituted by 1 - 3 independent $R^{4'}$'s [where $R^4$ and $R^{4'}$ independently represent an alkyl group having 1 - 6 carbon atoms which may be substituted by a fluorine atom, a halogen atom, a nitro group, a cyano group, $-A-R^{5A}$ {where A represents -CO-, $-CO_2-$, $-CONR^6-$, -O-, -OCO-, $-NR^6-$, $-NR^6CO-$, $-NR^6SO_2-$, $-NR^6CONH-$, $-NR^6CSNH-$ or $-NR^6COO-$ (where $R^6$ independently has the same meaning as the aforementioned $R^3$), and $R^{5A}$ represents a hydrogen atom, an alkyl group having 1 - 6 carbon atoms which may be substituted by a fluorine atom, or a cycloalkyl group having 3 - 7 carbon atoms}, or $-B-(CH_2)_n-R^{5B}$ {where B represents a single bond, -CO-, $-CO_2-$, $-CONR^{6'}-$, -O-, -OCO-, $-NR^{6'}-$, $-NR^{6'}CO-$, $-NR^{6'}SO_2-$, $-NR^{6'}CONH-$, $-NR^{6'}CSNH-$ or $-NR^{6'}COO-$ (where $R^{6'}$ independently has the same meaning as the aforementioned $R^3$), n represents an integer of 1 or 2, $R^{5B}$ represents an alkyl group having 1 - 6 carbon atoms which may be substituted by a fluorine atom, a cycloalkyl group having 3 - 7 carbon atoms, a halogen atom, a hydroxyl group, a cyano group, an alkoxy group having 1 - 5 carbon atoms, or $-NR^{7'}R^{8'}$ (where $R^{7'}$ and $R^{8'}$ independently have the same meaning as the aforementioned $R^3$)}], or $-C{\equiv}C-R^9$ {where $R^9$ represents a hydrogen atom, an alkyl group having 1 - 6 carbon atoms which may be substituted by a fluorine atom, a cycloalkyl group having 3 - 7 carbon atoms, or an aryl group which may be substituted by $R^{10}$ (where $R^{10}$ represents an alkyl group having 1 - 6 carbon atoms which may be substituted by a fluorine atom, or a halogen atom)}. The present invention also relates to a pharmaceutical and an androgen receptor agonist, each comprising the tetrahydroquinoline derivative of the formula (I) or pharmacologically acceptable salts thereof as the active ingredient.

## BRIEF DESCRIPTION OF THE DRAWINGS

FIG. 1 is a graph showing the effect of a compound of the present invention (the compound of Example 1) on the weight of the prostate as compared with DHT.

FIG. 2 is a graph showing the effect of a compound of the present invention (the compound of Example 1) on the femoral bone mineral density as compared with DHT.

FIG. 3 is a graph showing the effect of a compound of the present invention (the compound of Example 1) on the weight of the levator ani muscle as compared with DHT.

FIG. 4 is a graph showing the effect of compounds of the present invention (the compounds of Examples 8 and 23) on the weight of the prostate as compared with DHT.

FIG. 5 is a graph showing the effect of compounds of the present invention (the compounds of Examples 8 and 23) on the femoral bone mineral density as compared with DHT.

FIG. 6 is a graph showing the effect of compounds of the present invention (the compounds of Examples 8 and 23) on the weight of the levator ani muscle as compared with DHT.

FIG. 7 is a graph showing the effect of a compound of the present invention (the compound of Example 16) on the weight of the prostate.

FIG. 8 is a graph showing the effect of a compound of the present invention (the compound of Example 16) on the femoral bone mineral density.

Fig. 9 is a graph showing the effect of a compound of the present invention (the compound of Example 16) on the weight of the levator ani muscle.

## BEST MODE FOR CARRYING OUT THE INVENTION

According to another embodiment of the present invention, there are provided tetrahydroquinoline derivatives of the formula (I) where $R^1$ is a nitro group or a cyano group, i is 0, X is $-C(CH_3)_2-CH_2-$, Y is -NHCO- or -NHCONH-, and $R^2$ is a phenyl group which may be substituted by 1 - 3 independent $R^4$'s, or a heteroaryl group which may be substituted by 1 - 3 independent $R^{4'}$'s [where $R^4$ and $R^{4'}$ independently represent an alkyl group having 1 - 6 carbon atoms which may be substituted by a fluorine atom, a halogen atom, $-A-R^{5A}$ {where A represents -CO-, -O-, -OCO-, $-NR^6-$, $-NR^6CO-$ or $-NR^6CONH-$ (where $R^6$ represents a hydrogen atom or a methyl group), and $R^{5A}$ represents a hydrogen atom, an alkyl group having 1 - 6 carbon atoms which may be substituted by a fluorine atom, or a cycloalkyl group having 3 - 7 carbon atoms}, or $-B-(CH_2)_n-R^{5B}$ {where B represents -CO-, -O-, -OCO-, $-NR^{6'}-$, $-NR^{6'}CO-$ or $-NR^{6'}CONH-$(where $R^{6'}$ represents a hydrogen atom or a methyl group), n represents an integer of 1 or 2, and $R^{5B}$ represents an alkyl group having 1 - 6 carbon atoms which may be substituted by a fluorine atom, a cycloalkyl group having 3 - 7 carbon atoms, or an alkoxy group having 1 - 5 carbon atoms}; or pharmacologically acceptable salts thereof.

According to yet another embodiment of the present invention, there are provided tetrahydroquinoline derivatives of the formula (I) where $R^1$ is a nitro group or a cyano group, i is 0, X is $-C(CH_3)_2-CH_2-$, Y is -NHCO-, and $R^2$ is a phenyl group which may be substituted by 1 - 3 independent $R^4$'s, or a heteroaryl group which may be substituted by 1 - 3 independent $R^{4'}$'s [where $R^4$ and $R^{4'}$ independently represent an alkyl group having 1 - 6 carbon atoms which may be substituted by a fluorine atom, a halogen atom, $-A-R^{5A}$ {where A represents -CO-, -O-, -OCO-, -NH-, -NHCO- or -NHCONH-, and $R^{5A}$ represents a hydrogen atom, an alkyl group having 1 - 6 carbon atoms which may be substituted by a fluorine atom, or a cycloalkyl group having 3 - 7 carbon atoms}, or $-B-(CH_2)_n-R^{5B}$ {where B represents -CO-, -O-, -OCO-, -NH-, -NHCO- or -NHCONH-, n represents an integer of 1 or 2, and $R^{5B}$ represents an alkyl group having 1 - 6 carbon atoms which may be substituted by a fluorine atom, a cycloalkyl group having 3 - 7 carbon atoms, or an alkoxy group having 1 - 5 carbon atoms}; or pharmacologically acceptable salts thereof.

According to a further embodiment of the present invention, there are provided tetrahydroquinoline derivatives of the formula (I) where $R^1$ is a nitro group or a cyano group, i is 0, X is $-C(CH_3)_2-CH_2-$, Y is -NHCO-, and $R^2$ is a heteroaryl group which may be substituted by 1 - 3 independent $R^{4'}$'s {where $R^{4'}$ represents an alkyl group having 1 - 6 carbon atoms which may be substituted by a fluorine atom, a halogen atom, $-A-R^{5A}$ (where A represents -O- or -NHCO-, and $R^{5A}$ represents a hydrogen atom, or an alkyl group having 1 - 6 carbon atoms which may be substituted by a fluorine atom), or $-B-CH_2-R^{5B}$ (where B represents -O- or -NHCO-, and $R^{5B}$ represents an alkyl group having 1 - 6 carbon atoms which may be substituted by a fluorine atom, or an alkoxy group having 1 - 5 carbon atoms)}; or pharmacologically acceptable salts thereof.

According to a yet further embodiment of the present invention, there are provided tetrahydroquinoline derivatives of the formula (I) where $R^1$ is a nitro group or a cyano group, i is 0, X is $-C(CH_3)_2-CH_2-$, Y is -NHCO-, and $R^2$ is a phenyl group having substituent $R^4$ at 4-position, or a 6-membered heteroaryl group having substituent $R^{4'}$ at 4-position [where $R^4$ and $R^{4'}$ independently represent a halogen atom, $-O-R^{5A}$, or $-NHCO-R^{5A}$ (where $R^{5A}$ represents a hydrogen atom, or an alkyl group having 1 - 6 carbon atoms which may be substituted by a fluorine atom)]; or pharmacologically acceptable salts thereof.

According to a still further embodiment of the present invention, there are provided tetrahydroquinoline derivatives of the formula (I) where $R^1$ is a nitro group or a cyano group, i is 0, X is $-C(CH_3)_2-CH_2-$, Y is -NHCO-, and $R^2$ is a phenyl group having substituent $R^4$ at 4-position, or a 6-membered heteroaryl group having substituent $R^{4'}$ at 4-position [where $R^4$ and $R^{4'}$ independently represent $-NHCO-R^{5A}$ (where $R^{5A}$ represents a hydrogen atom, or an alkyl group having 1 - 6 carbon atoms which may be substituted by a fluorine atom)]; or pharmacologically acceptable salts thereof.

According to an additional embodiment of the present invention, there are provided tetrahydroquinoline derivatives of the formula (I) where $R^1$ is a nitro group or a cyano group, i is 0, X is $-C(CH_3)_2-CH_2-$, Y is -NHCO-, and $R^2$

represents $-C\equiv C-R^9$ {where $R^9$ represents an alkyl group having 1 - 6 carbon atoms which may be substituted by a fluorine atom, or an aryl group which may be substituted by $R^{10}$ (where $R^{10}$ represents an alkyl group having 1 - 6 carbon atoms which may be substituted by a fluorine atom, or a halogen atom)}; or pharmacologically acceptable salts thereof.

According to a still additional embodiment of the present invention, there are provided tetrahydroquinoline derivatives of the formula (I) where $R^1$ is a nitro group or a cyano group, i is 0, X is $-C(CH_3)_2-CH_2-$, Y is -NHCO-, and $R^2$ represents $-C\equiv C-R^9$ (where $R^9$ represents an alkyl group having 1 - 6 carbon atoms which may be substituted by a fluorine atom, or a phenyl group); or pharmacologically acceptable salts thereof.

The substituents in the formula (I) representing the compounds of the present invention will be described below.

Specific examples of the "alkyl group having 1 - 6 carbon atoms" include straight chain or branched chain alkyl groups, such as a methyl group, an ethyl group, a *n*-propyl group, an isopropyl group, a n-butyl group, an isobutyl group, a *tert*-butyl group, a sec-butyl group, a n-pentyl group, a *tert*-amyl group, a 3-methylbutyl group, a neopentyl group, a *n*-hexyl group, a 3,3-dimethylbutyl group, and a 2-ethylbutyl group.

Specific examples of the "cycloalkyl group having 3 - 7 carbon atoms" include a cyclopropyl group, a cyclobutyl group, a cyclopentyl group, a cyclohexyl group, and a cycloheptyl group.

Specific examples of "alkylene group having 1 - 5 carbon atoms" include a methylene group, an ethylene group, a propylene group, a tetramethylene group, and a pentamethylene group.

Specific examples of the "aralkyl group having 7 - 9 carbon atoms" include a benzyl group, a phenethyl group, and a phenylpropyl group.

Specific examples of the "heteroaryl group" include a 2-pyridyl group, a 3-pyridyl group, a 4-pyridyl group, a 2-pyrazinyl group, a 2-pyrrolyl group, a 2-indolyl group, a 2-furyl group, a 3-furyl group, a 2-thienyl group, a 3-thienyl group, a 2-pyrrole group, and a 3-pyrrole group. Specific examples of the "6-membered heteroaryl group" include a 2-pyridyl group, a 3-pyridyl group, a 4-pyridyl group, and a 2-pyrazinyl group.

Referring to the "alkyl group having 1 - 6 carbon atoms which may be substituted by a fluorine atom", specific examples of the fluorine atom substituted alkyl group include a difluoromethyl group, a trifluoromethyl group, a 2,2,2-trifluoroethyl group, and a tetrafluoroethyl group.

Specific examples of the "halogen atom" include a fluorine atom, a chlorine atom, a bromine atom, and an iodine atom.

Specific examples of the "alkoxy group having 1 - 5 carbon atoms" include straight chain or branched chain alkoxy groups, such as a methoxy group, an ethoxy group, a *n*-propoxy group, an isopropoxy group, a *n*-butoxy group, an isobutoxy group, a *tert*-butoxy group, a *sec*-butoxy group, a *n*-pentyloxy group, a *tert*-amyloxy group, a 3-methylbutoxy group, and a neopentyloxy group.

Specific examples of the "aryl group" include a phenyl group, and a naphthyl group (a 1-naphthyl group and a 2-naphthyl group).

Preferred modes for the compounds of the formula (I) include, for example, the following:

$R^1$ is preferably a cyano group, and is preferably substituted at 9-position when i is 1 (at 8-position when i is 0).
i is preferably 0.

In the definition of X, the number of the substituents by which the alkylene group having 1 - 5 carbon atoms may be substituted is preferably 1 - 3, and the preferred examples of the substituent include a methyl group and an ethyl group. The preferred example of X is $-C(CH_3)_2-CH_2-$.

Y is preferably -NHCO-, $-NHSO_2-$, or -NHCONH-, more preferably -NHCO- or -NHCONH-, and even more preferably -NHCO-.

In the definition of $R^2$, the phenyl group which may be substituted by 1 - 3 independent $R^4$'s is more preferably a phenyl group which may be substituted by 1 - 2 independent $R^4$'s, and is even more preferably a phenyl group having substituent $R^4$ at 4-position when the position of binding to Y is numbered as 1-position.

The preferred examples of the substituent $R^4$ are a halogen atom, an alkyl group having 1 - 6 carbon atoms which may be substituted by a fluorine atom, $-A-R^{5A}$ {where A represents -CO-, -O-, -OCO-, $-NR^6-$, $-NR^6CO-$ or $-NR^6CONH-$(where $R^6$ represents a hydrogen atom or a methyl group), and $R^{5A}$ represents a hydrogen atom, an alkyl group having 1 - 6 carbon atoms which may be substituted by a fluorine atom, or a cycloalkyl group having 3 - 7 carbon atoms}, and $-B-(CH_2)_n-R^{5B}$ {where B represents -CO-, -O-, -OCO-, $-NR^{6'}-$, $-NR^{6'}CO-$ or $-NR^{6'}CONH-$ (where $R^{6'}$ represents a hydrogen atom or a methyl group), n represents an integer of 1 or 2, and $R^{5B}$ represents an alkyl group having 1 - 6 carbon atoms which may be substituted by a fluorine atom, a cycloalkyl group having 3 - 7 carbon atoms, or an alkoxy group having 1 - 5 carbon atoms}.

With reference to $R^4$, examples of "the alkyl group having 1 - 6 carbon atoms which may be substituted by a fluorine atom" are a methyl group, and a trifluoromethyl group.

Examples of "-A-$R^{5A}$" are as follows:

As -O-$R^{5A}$ (where $R^{5A}$ represents a hydrogen atom, or an alkyl group having 1 - 6 carbon atoms which may be substituted by a fluorine atom), there are named, for example, -OMe, -OEt, -$OPr^n$, -$OPr^i$, -$OBu^n$, -$OCHF_2$, -$OCF_3$, and -$OCH_2CF_3$.

As -CO-$R^{5A}$, -CO-($C_1$-$C_6$ alkyl group)(e.g., -COMe), for example, is named.

As -OCO-$R^{5A}$, -OCO-($C_1$-$C_6$ alkyl group)(e.g., -OCOMe), for example, is named.

The substituent -$NR^6$-$R^{5A}$ is preferably -NH-$R^{5A}$, its examples being -$NH_2$ and -$NMe_2$.

The substituent -$NR^6$CO-$R^{5A}$ is preferably -NHCO-$R^{5A}$, its examples being -NHCOH, and -NHCO-($C_1$-$C_6$ alkyl group)(e.g., -NHCOMe).

The substituent -$NR^6$CONH-$R^{5A}$ is preferably -NHCONH-$R^{5A}$, its example being -NHCONH-($C_1$-$C_6$ alkyl group)(e.g., -NHCONHMe).

Examples of "-B-$(CH_2)_n$-$R^{5B}$" are -O-$(CH_2)_n$-($C_1$-$C_5$ alkoxy group) (where n represents an integer of 1 or 2) (for example, -$OCH_2$OMe and -$OCH_2CH_2$OMe).

The more preferred examples of the substituent $R^4$ are an alkyl group having 1 - 6 carbon atoms which may be substituted by a fluorine atom, a halogen atom, and -A-$R^{5A}$ {where A represents -CO-, -O-, -OCO-, -NH-, -NHCO- or -NHCONH-, and $R^{5A}$ represents a hydrogen atom, an alkyl group having 1 - 6 carbon atoms which may be substituted by a fluorine atom, or a cycloalkyl group having 3 - 7 carbon atoms}, further preferably, a halogen atom, -O-$R^{5A}$ and -NHCO-$R^{5A}$ (where $R^{5A}$ represents a hydrogen atom, or an alkyl group having 1 - 6 carbon atoms which may be substituted by a fluorine atom), even more preferably, -NHCO-$R^{5A}$ (where $R^{5A}$ represents a hydrogen atom, or an alkyl group having 1 - 6 carbon atoms which may be substituted by a fluorine atom), and particularly, -NHCOH, and -NH-CO-($C_1$-$C_6$ alkyl group).

In the definition of $R^2$, the heteroaryl group (preferably, a pyridyl group and a furyl group) which may be substituted by 1 - 3 independent $R^{4'}$'s is more preferably a heteroaryl group which may be substituted by 1 - 2 independent $R^{4'}$'s, and when $R^2$ is 6-membered heteroaryl (preferably, a pyridyl group), the heteroaryl group is even more preferably a heteroaryl group having substituent $R^{4'}$ at 4-position when the position of binding to Y is numbered as 1-position.

The preferred examples of the substituent $R^{4'}$ are the same as the preferred examples of the substituent $R^4$ described earlier.

In the definition of $R^2$, the substituent $R^9$ of -C≡C-$R^9$ is preferably an alkyl group having 1 - 6 carbon atoms which may be substituted by a fluorine atom, or an aryl group which may be substituted by $R^{10}$ (where $R^{10}$ represents an alkyl group having 1 - 6 carbon atoms which may be substituted by a fluorine atom, or a halogen atom). More preferably, the substituent $R^9$ is an alkyl group having 1 - 6 carbon atoms which may be substituted by a fluorine atom, or a phenyl group, its examples being a methyl group, an ethyl group, a *n*-propyl group, a *n*-butyl group, a n-pentyl group, a *n*-hexyl group, and a phenyl group.

The preferred compounds of the present invention are as follows:

*N*-[2-(8-Cyano-2,3,3a,4,5,9b-hexahydro-1*H*-cyclopenta[c]quinolin-4-yl)-2-methylpropyl]-4-trifluoromethoxybenzamide

*N*-[2-(8-Cyano-2,3,3a,4,5,9b-hexahydro-1*H*-cyclopenta[c]quinolin-4-yl)-2-methylpropyl]-4-(2,2,2-trifluoroethoxy) benzamide

*N*-[2-(8-Cyano-2,3,3a,4,5,9b-hexahydro-1*H*-cyclopenta[*c*]quinolin-4-yl)-2-methylpropyl]-4-formylaminobenzamide

6-Acetamido-*N*-[2-(8-cyano-2,3,3a,4,5,9b-hexahydro-1*H*-cyclopenta[*c*]quinolin-4-yl)-2-methylpropyl]nicotinamide

If asymmetric carbon is present in the compound of the present invention represented by the formula (I), its racemic compounds, diastereomers, and individual optical isomers are all included in the present invention. If its geometrical isomers are present, (E) compounds, (Z) compounds, and mixtures of them are all included in the present invention.

Since the tetrahydroquinoline ring of the compound of the present invention represented by the formula (I) has three asymmetric carbon atoms, diastereomers are present in the compounds of the present invention. Their relative configuration is preferably (3a*R**,4*S**,9b*S**) when i is 0, and (4a*R**,5*S**,10b*S**) when i is 1.

The preferred isomers as the compounds of the present invention are as follows:

(3a*R**,4*S**,9b*S**)-*N*-[2-(8-Cyano-2,3,3a,4,5,9b-hexahydro-1*H*-cyclopenta[*c*]quinolin-4-yl)-2-methylpropyl]-4-trifluoromethoxybenzamide (compound of Example 1)

(3aR*,4S*,9bS*)-*N*-[2-(8-Cyano-2,3,3a,4,5,9b-hexahydro-1*H*-cyclopenta[*c*]quinolin-4-yl)-2-methylpropyl]-4-(2,2,2-trifluoroethoxy)benzamide (compound of Example 8)
(3aR*,4S*,9bS*)-*N*-[2-(8-Cyano-2,3,3a,4,5,9b-hexahydro-1*H*-cyclopenta[*c*]quinolin-4-yl)-2-methylpropyl]-4-formylaminobenzamide (compound of Example 16)
(3a*R**,4*S**,9b*S**)-6-Acetamido-*N*-[2-(8-cyano-2,3,3a,4,5,9b-hexahydro-1*H*-cyclopenta[*c*]quinolin-4-yl)-2-methylpropyl]nicotinamide (compound of Example 23)

The salts of the compounds of the present invention represented by the formula (I) are not limited, as long as they are those which are pharmacologically acceptable. Their examples include hydrohalogenic acid salts, such as hydrofluorides, hydrochlorides, hydrobromides and hydroiodides, inorganic acid salts, such as nitrates, perchlorates, sulfates, phosphates and carbonates, lower alkylsulfonic acid salts, such as methanesulfonates, trifluoromethanesulfonates and ethanesulfonates, arylsulfonic acid salts, such as benzenesulfonates and p-toluenesulfonates, carboxylic acid salts, such as acetates, fumarates, succinates, citrates, tartrates, oxalates and maleates, amino acid salts, such as glycine salts, alanine salts, glutamates and aspartates, and alkali metal salts, such as sodium salts and potassium salts.
Solvates of the compounds of the present invention are also included in the present invention. Examples of the solvates are solvates with solvents, such as acetone, 2-butanol, 2-propanol, ethanol, ethyl acetate, tetrahydrofuran and diethyl ether.
The tetrahydroquinoline derivatives of the present invention can be produced by the following methods:

[Production Method 1]

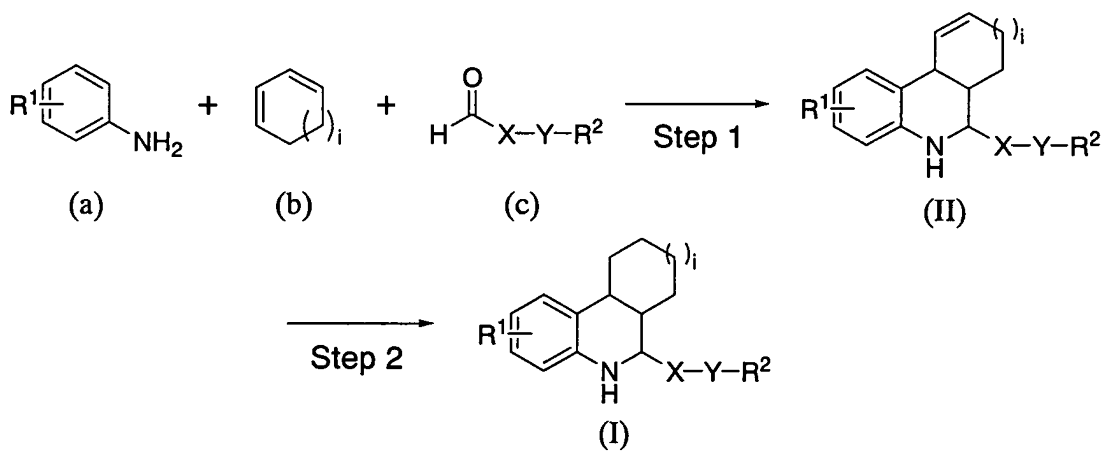


where all the symbols are as defined above.
The compound of the present invention, represented by the formula (I), can be produced by the reactions of Steps 1 and 2 described below.

(Step 1) In this step, the compound of the formula (II) can be produced by reacting the compounds represented by the formulas (a), (b) and (c) in an inert solvent in the presence or absence of an acid.

The compounds represented by the formulas (a), (b) and (c) can be obtained as commercially available reagents, or by easy derivation therefrom by routine chemical reactions.

The present reaction will be described concretely. Any types of acids, organic or inorganic, are preferred. For example, acetic acid, trifluoroacetic acid, p-toluenesulfonic acid, hydrochloric acid, sulfuric acid, tin tetrachloride, titanium tetrachloride, boron trifluoride diethyl etherate, diethylaluminum chloride, or ethylaluminum dichloride is used. The acid is preferably used in an amount of a catalytic amount to 10 equivalents with respect to the compound represented by the formula (a). The reaction solvent is not limited, as long as it is a solvent which does not markedly impede the present reaction. The preferred reaction solvent is dichloromethane, chloroform, 1,2-dichloroethane, hexane, benzene, toluene, dioxane, tetrahydrofuran, acetonitrile, methanol, ethanol, water or a mixture of these solvents. The reaction temperature is preferably -20 to 100°C, and the reaction time is preferably 5 minutes to 48

hours.

(Step 2) In this step, the compound of the formula (I) can be produced by catalytically reducing the compound represented by the formula (II) in the presence of a catalyst.

The present reaction will be described concretely. Examples of the catalyst are 5% palladium carbon, 10% palladium carbon, 30% palladium carbon, platinum oxide, and Wilkinson's catalyst. The amount of the catalyst is preferably 1/10 of, to an equal weight to, the weight of the compound represented by the formula (II), and the hydrogen pressure is preferably 1 to 5 atmospheres. The reaction solvent is not limited, as long as it is a solvent which does not markedly impede the present reaction. The preferred reaction solvent is methanol, ethanol, ethyl acetate, or tetrahydrofuran. The reaction temperature is preferably 25 to 70°C, and the reaction time is preferably 30 minutes to 72 hours.

[Production Method 2]

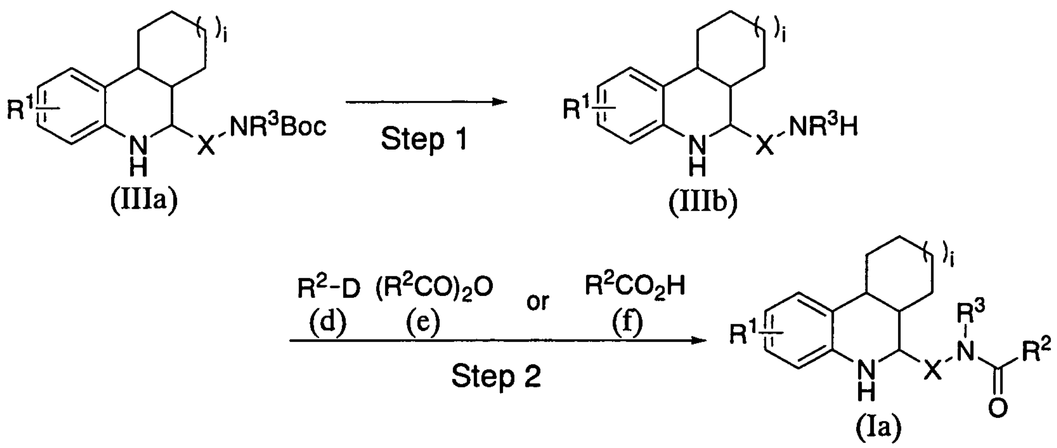


where all the symbols are as defined above, Boc signifies a *tert*-butoxycarbonyl group, and D signifies a halogenated carbonyl group.

The compound of the present invention, represented by the formula (Ia), can be produced by the reactions of Steps 1 and 2 described below, in addition to Production Method 1.

(Step 1) In this step, the compound represented by the formula (IIIa), which can be produced by the same method as in Step 1 of Production Method 1, is treated with an acid for deprotection, whereby the compound represented by the formula (IIIb) can be produced.

The present reaction will be described concretely. Any types of acids, organic or inorganic, are preferred. For example, acetic acid, trifluoroacetic acid, p-toluenesulfonic acid, hydrochloric acid, or sulfuric acid is named. The acid is preferably used in an amount of 1 to 50 equivalents with respect to the compound represented by the formula (IIIa). The reaction solvent is not limited, as long as it is a solvent which does not markedly impede the present reaction. The preferred reaction solvent is dichloromethane, chloroform, 1,2-dichloroethane, hexane, benzene, toluene, dioxane, tetrahydrofuran, acetonitrile, methanol, ethanol, water or a mixture of these solvents. The reaction temperature is preferably 0 to 100°C, and the reaction time is preferably 30 minutes to 24 hours.

(Step 2) In this step, the compound represented by the formula (Ia) can be produced by reacting the compound represented by the formula (IIIb) with the compound represented by the formula (d), (e) or (f) without a solvent or in an inert solvent in the presence or absence of a base to form an amide bond.

Specific examples of the "halogenated carbonyl group" as D are a chlorocarbonyl group and a bromocarbonyl group.

The reaction for forming the amide bond is known, and is performed, for example, by a method using an acid halide or an acid anhydride, or a method using a condensing agent.

The reactions of these methods will be described concretely. The method using an acid halide or an acid anhydride is a method designed to react the compound represented by the formula (d) or (e) with the compound represented by the formula (IIIb) without a solvent or in an inert solvent in the presence or absence of a base, thereby

amidating the compound of the formula (IIIb). The base is preferably a tertiary amine, its examples being triethylamine and pyridine. The compound represented by the formula (d) or (e) is used preferably in an amount of 1 to 10 equivalents with respect to the compound represented by the formula (IIIb). The base is preferably used in an amount of from 1 equivalent to a large excess with respect to the acid halide or acid anhydride. The reaction solvent is not limited, as long as it is a solvent which does not markedly impede the present reaction. The preferred reaction solvent is N,N-dimethylformamide, dichloromethane, chloroform, 1,2-dichloroethane, 1,1,2,2-tetrachloroethane, toluene, and pyridine. The reaction temperature is preferably 0 to 80°C, and the reaction time is preferably 30 minutes to 24 hours.

The method using a condensing agent is a method which reacts the compound represented by the formula (IIIb) with the compound represented by the formula (f) with the use of a condensing agent, such as 2-chloro-1,3-dimethylimidazolinium chloride, dicyclohexylcarbodiimide, or 1-ethyl-3-(3-dimethylaminopropyl)carbodiimide, without a solvent or in an inert solvent in the presence or absence of a base. The condensing agent is preferably used in an amount of 1 to 2 equivalents with respect to the compound represented by the formula (f). The base is preferably a tertiary amine, its examples being 4-methylmorpholine, triethylamine and pyridine. The base is preferably used in an amount of from 1 equivalent to a large excess with respect to the compound represented by the formula (f). The reaction solvent is not limited, as long as it is a solvent which does not markedly impede the present reaction. The preferred reaction solvent is N,N-dimethylformamide, dimethyl sulfoxide, tetrahydrofuran, dioxane, dichloromethane, chloroform, and 1,2-dichloroethane. The reaction temperature is preferably 0 to 150°C, and the reaction time is preferably 1 to 48 hours.

[Production Method 3]

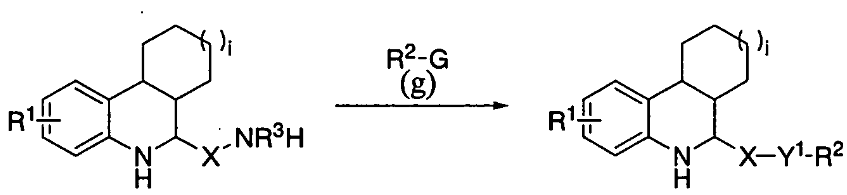


(IIIb) (Ib)

where all the symbols are as defined above, $Y^1$ represents $-NR^3SO_2$, $-NR^3CONH-$, or $-NR^3CSNH-$, and G signifies a chlorosulfonyl group, an isocyanato group, or an isothiocyanato group.

The compound of the present invention, represented by the formula (Ib), can be produced by the reaction described below, in addition to Production Method 1.

In the present reaction, the compound represented by the formula (Ib) can be produced by reacting the compound represented by the formula (IIIb) with the compound represented by the formula (g) without a solvent or in an inert solvent in the presence or absence of a base.

The present reaction will be described concretely. The base is preferably a tertiary amine, its examples being triethylamine and pyridine. The compound represented by the formula (g) is used preferably in an amount of 1 to 10 equivalents with respect to the compound represented by the formula (IIIb). The base is preferably used in an amount of from 1 equivalent to a large excess with respect to the compound represented by the formula (g). The reaction solvent is not limited, as long as it is a solvent which does not markedly impede the present reaction. The preferred reaction solvent is N,N-dimethylformamide, dichloromethane, chloroform, 1,2-dichloroethane, 1,1,2,2-tetrachloroethane, toluene, and tetrahydrofuran. The reaction temperature is preferably 0 to 80°C, and the reaction time is preferably 30 minutes to 12 hours.

The compounds of the present invention, which are produced by the above-described production methods, are isolated and purified as free compounds, their salts, their hydrates, various solvates thereof, such as ethanolates, or crystalline polymorphic substances. The pharmacologically acceptable salts of the compounds according to the present invention can be produced by the general salt-forming reaction. The isolation and purification are performed by applying chemical operations, such as extractive fractionation, crystallization, and various chromatographic techniques. The stereochemically pure optical isomers can be obtained by using suitable starting compounds, or by racemic resolution of racemic compounds.

The tetrahydroquinoline derivatives of the present invention or their salts have an excellent AR agonistic action. These substances can be used as active ingredients to form pharmaceuticals or AR agonists, which can be widely applied to the prevention or treatment of various AR-related diseases.

The AR agonistic action can be expected to cure the diseases described below.

Diseases which can be expected to be cured by the anabolic action are as follows: Their examples to be treated because of potent action on bone tissues are primary osteoporosis (senile, postmenopausal and juvenile osteoporosis) and secondary osteoporosis [osteoporosis ascribed to hyperthyroidism, Cushing syndrome (due to steroid treatment), acromegaly, hypogonadism, dysosteogenesis, hypophosphatasemia or diabetes, or disuse osteoporosis]. Examples of the diseases to be treated because of potent action on muscular tissues are wasting diseases ascribed to postoperative condition, malignant tumor, trauma, chronic renal disease, burns or AIDS. Examples of the diseases to be treated because of erythropoiesis promoting action are hematopoietic dysfunction and diseases related thereto, for instance, aplastic anemia, hemolytic anemia, sickle-cell anemia, idiopathic thrombocytopenic purpura, myelofibrosis and renal anemia.

Diseases which can be expected to be cured by the sexual action include, for example, male hypogonadism, male sexual dysfunction (impotence, dysspermatogenic sterility), abnormal sex differentiation (male hermaphroditism), male delayed puberty, carcinoma of female genitalia (including pain associated with carcinoma), breast cancer, mastopathy, endometriosis and female sexual dysfunction.

The pharmaceuticals of the present invention can be applied widely to these AR-related diseases, and can be applied to diseases which are not exemplified here, if the modulation of AR function is required for them at present or in the future.

The pharmaceuticals of the present invention can be administered orally or parenterally, and may be administered systemically or administered locally.

Their dosage forms are not limited, and can be selected, as desired, according to the route of administration. Their examples include tablets, capsules, sugar-coated tablets, granules, fine granules, inhalations, suppositories, liquids and solutions, syrups, dry syrups, suspensions, emulsions, lotions, ointments, patches, sprays, gels, nasal drops, eye drops, and injections.

These preparations can be produced by incorporating organic or inorganic, solid or liquid vehicles, adjuvants, stabilizers, wetting agents, emulsifying agents, buffers, and various other pharmacologically acceptable additives.

The dose of the pharmaceutical of the present invention in humans is determined, as desired, according to various conditions, such as the purpose of treatment or prevention, the patient's sex, body weight, age, and health condition, the type and severity of the disease, dosage form, the route of administration, and the duration of administration. The daily dose of the tetrahydroquinoline derivative of the present invention is generally 0.01 to 100 mg/kg.

The pharmaceuticals of the present invention may be used in the treatment of androgen receptor-mediated diseases in warm-blooded animals, such as domestic animals, pets, bred animals, or wild animals. The dosage forms and doses in this case can be determined by reference to the dosage forms and doses in humans.

Examples

The compounds of the present invention and the methods for their production will be described in further detail by working examples. However, the present invention is not to be interpreted restrictedly because of these descriptions.

$^1$H-NMR spectra were recorded on JNM-EX270 Spectrometer (270 MHz, JEOL Ltd.), with tetramethylsilane (TMS) as an internal standard. δ values are expressed in ppm.

In the structural formulas and tables offered below, Me represents a methyl group, Et an ethyl group, Pr a propyl group, Bu a butyl group, and Hex a hexyl group.

[Example 1] Production of (3a*R**,4*S**,9b*S**)-*N*-[2-(8-cyano-2,3,3a,4,5,9b-hexahydro-1*H*-cyclopenta[*c*]quinolin-4-yl)-2-methylpropyl]-4-trifluoromethoxybenzamide

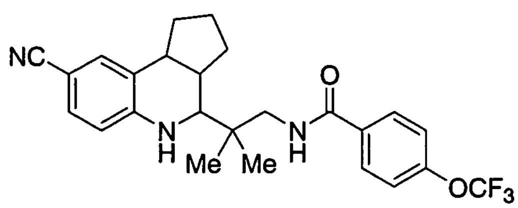

(For convenience's sake, the absolute configuration in the chemical formula is omitted. The same will be true of the chemical formulas shown below.)

(1) (3a*R**,4*S**,9b*S**)-[2-(8-Cyano-3a,4,5,9b-tetrahydro-1*H*-cyclopenta[c]quinolin-4-yl)-2-methylpropyl]carbamate *tert*-butyl ester

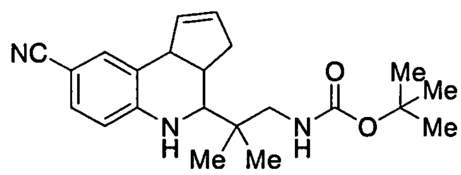

4-Aminobenzonitrile (50.0 g), 41.8 mL of cyclopentadiene, and 85.0 g of 2,2-dimethyl-3-oxopropylcarbamate *tert*-butyl ester were dissolved in 1,000 mL of acetonitrile, and 3.2 mL of trifluoroacetic acid was added at 0°C. After overnight stirring at room temperature, crystals precipitated were collected by filtration to obtain 50.4 g of the captioned compound. Its physical properties are shown below.
$^1$H-NMR ($CDCl_3$) δ values: 0.99 (3H, s), 1.01 (3H, s), 1.35 (9H, s), 2.21-2.30 (1H, m), 2.45-2.55 (1H, m), 2.81-2.92 (2H, m), 3.27 (1H, brs), 3.38-3.42 (1H, m), 3.98 (1H, d, J = 7.9 Hz), 4.69 (1H, brs), 5.74-5.76 (1H, m), 5.85-5.88 (1H, m), 6.64 (1H, d, J = 7.9 Hz), 7.17 (1H, d, J = 7.9 Hz), 7.19 (1H, s).

(2) (3a*R**,4*S**,9b*S**)-[2-(8-Cyano-2,3,3a,4,5,9b-hexahydro-1H-cyclopenta[c]quinolin-4-yl)-2-methylpropyl]carbamate *tert*-butyl ester

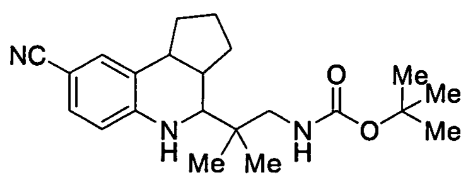

The compound (10.0 g) obtained in (1) was dissolved in 750 mL of ethyl acetate, 100 mg of platinum oxide was added, and the mixture was stirred for 1 hour at room temperature in an atmosphere of hydrogen. The platinum oxide was removed by filtration, and the solvent was distilled off under reduced pressure to obtain 10.4 g of the captioned compound. Its physical properties are shown below.
$^1$H-NMR ($CDCl_3$) δ values: 0.97 (3H, s), 0.99 (3H, s), 1.37 (9H, s), 1.43-1.69 (5H, m), 1.81-1.98 (2H, m), 2.37 (1H, q, J = 7.9, 12.5 Hz), 2.90 (1H, dd, J = 5.3, 14.2 Hz), 3.24-3.34 (3H, m), 4.68 (1H, brs), 6.53 (1H, d, J = 8.6 Hz), 7.19 (1H, dd, J = 1.7, 8.6 Hz), 7.32 (1H, d, J =1.7 Hz).

(3) (3a*R**,4*S**,9b*S**)-4-(2-Amino-1,1-dimethylethyl)-2,3,3a,4,5,9b-hexahydro-1*H*-cyclopenta[*c*]quinoline-8-carbonitrile

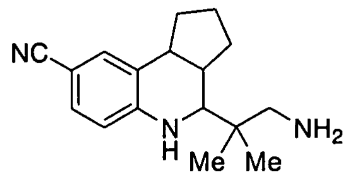

The compound (3.0 g) obtained in (2) was dissolved in 10 mL of tetrahydrofuran, and 10 mL of a 4N hydrochloric acid-dioxane solution was added. The mixture was stirred for 2 hours at 50°C, whereafter an aqueous solution of 2N sodium hydroxide and ethyl acetate were added. The ethyl acetate layer was washed with a saturated aqueous solution of sodium chloride and water, and dried over anhydrous magnesium sulfate. Then, the solvent was distilled off under reduced pressure to obtain 1.5 g of the captioned compound. Its physical properties are shown below.
$^1$H-NMR ($CDCl_3$) δ values: 0.93 (3H, s), 1.03 (3H, s), 1.44-1.73 (4H, m), 1.81-1.91 (2H, m), 2.27-2.32 (1H, m), 2.72 (1H, dd, J = 12.5, 33.0 Hz), 3.25 (1H, t, J = 5.9 Hz), 3.44 (1H, d, J = 2.3 Hz), 6.44 (1H, d, J = 8.6 Hz), 7.00 (1H, brs), 7.15 (1H, dd, J = 1.7, 8.4 Hz), 7.29 (1H, s).

(4) (3a*R**,4*S**,9b*S**)-*N*-[2-(8-Cyano-2,3,3a,4,5,9b-hexahydro-1*H*-cyclopenta[*c*]quinolin-4-yl)-2-methylpropyl]-4-trifluoromethoxybenzamide

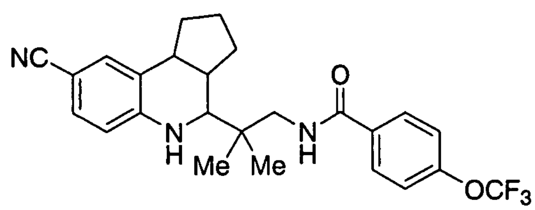

The compound (3.6 g) obtained in (3), and 2.8 g of 4-trifluoromethoxybenzoic acid were dissolved in 100 mL of N,N-dimethylformamide, and 3.4 mL of 4-methylmorpholine, 2.4 g of N-hydroxybenzotriazole and 3.4 g of 1-ethyl-3-(3-dimethylaminopropyl)carbodiimide hydrochloride were added. After the mixture was stirred for 2 hours at room temperature, water and ethyl acetate were added. The ethyl acetate layer was washed with water, and then dried over anhydrous magnesium sulfate. Then, the solvent was distilled off under reduced pressure. The residue was purified by silica gel column chromatography (elution solvent: hexane:ethyl acetate = 3:1) to obtain 3.2 g of the captioned compound. Its physical properties are shown below.
$^1$H-NMR ($CDCl_3$) δ: 1.08 (3H, s), 1.11 (3H, s), 1.52-1.74 (5H, m), 1.85-1.99 (1H, m), 2.42 (1H, q, J = 7.6 Hz), 3.19-3.27 (2H, m), 3.33 (1H, d, J = 2.3 Hz), 3.74 (1H, dd, J = 7.3, 14.2 Hz), 6.37 (1H, brs), 6.62 (1H, d, J = 8.6 Hz), 7.21-7.33 (4H, m), 7.77 (2H, d, J = 8.9Hz).
Compounds shown in Examples 2 to 29 were produced in the same manner as in Example 1. The physical properties of the resulting compounds are shown in Table 1.

Table 1

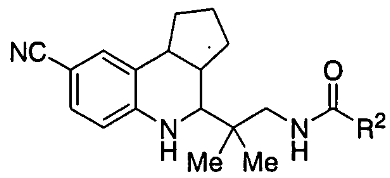

| Ex. No. | $R^2$ | $^1$H-NMR |
|---|---|---|
| 2 | 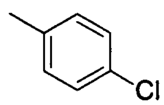 | ($CDCl_3$)δ: 1.08(3H, s), 1.11(3H, s), 1.30-1.74(4H, m), 1.84-2.01(2H, m), 2.88-2.96(1H, m), 3.19-3.26(2H, m), 3.33(1H, d, J=2.0Hz), 3.74(1H, dd, J=7.3, 14.2Hz), 6.41(1H, t, J=5.9Hz), 6.62(1H, d, J=8.2Hz), 7.22(1H, dd, J=2.0, 8.2Hz), 7.33(1H, s), 7.40(2H, d, J=8.6Hz), 7.67(2H, d, J=8.6Hz). |
| 3 | 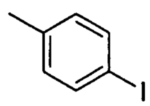 | ($CDCl_3$) δ: 1.07(3H, s), 1.10(3H, s), 1.52-1.74(4H, m), 1.84-1.96(2H, m), 2.39-2.42(1H, m), 3.18-3.25(2H, m), 3.32(1H, d, J=2.3Hz), 3.71(1H, dd, J=7.3, 14.2Hz), 6.38(1H, t, J=6.6Hz), 6.59(1H, d, J=8.6Hz), 7.22(1H, dd, J=1.7, 8.2Hz), 7.32(1H, brs), 7.44(2H, d, J=8.6Hz), 7.78(2H, d, J=8.6Hz). |
| 4 | 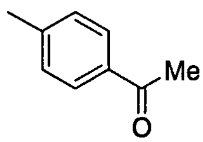 | ($CDCl_3$) δ: 1.08(3H, s), 1.12(3H, s), 1.42-1.75(4H, m), 1.84-1.99(2H, m), 2.41-2.43(1H, m), 2.63(3H, s), 3.26(2H, dd, J=6.3, 14.2Hz), 3.34(1H, d, J=2.3Hz), 3.74(1H, dd, J=7.3, 14.2Hz), 4.66(1H, brs), 6.51(1H, t, J=6.6Hz), 6.60(1H, d, J=8.3Hz), 7.21(1H, dd, J=2.0, 8.4Hz), 7.32(1H, s), 7.80(2H, d, J=8.6Hz), 8.00(2H, d, J=8.6Hz). |
| 5 | 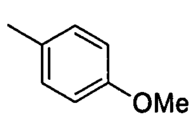 | ($CDCl_3$) δ: 1.06(3H, s), 1.10(3H, s), 1.41-1.74(4H, m), 1.84-1.97(2H, m), 2.36-2.41(1H, m), 3.18(1H, dd, J=5.9, 14.4Hz), 3.22-3.25(1H, m), 3.33(1H, d, J=2.0Hz), 3.74(1H, dd, J=7.6, 14.2Hz), 3.84(3H, s), 6.31(1H, t, J=6.6Hz), 6.61(1H, d, J=8.2Hz), 6.91(1H, d, J=8.9Hz), 7.21(1H, dd, J=1.6, 8.4Hz), 7.31(1H, s), 7.69(1H, d, J=8.9Hz). |
| 6 | 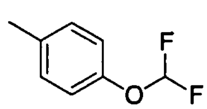 | ($CDCl_3$) δ: 1.07(3H, s), 1.11(3H, s), 1.48-1.80(4H, m), 1.84-1.98(2H, m), 2.37-2.45(1H, m), 3.18-3.26(2H, m), 3.33(1H, d, J=2.0Hz), 3.73(1H, dd, J=7.6, 14.3Hz), 4.68(1H, brs), 6.35(1H, t, J=6.6Hz), 6.56(1H, m), 6.59(1H, d, J=8.6Hz), 7.16(1H, d, J=8.9Hz), 7.21(1H, dd, J=1.6, 8.4Hz), 7.32(1H, s), 7.74(1H, d, J=8.9Hz). |

| | | |
|---|---|---|
| 7 | 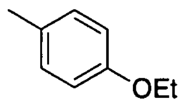 | ($CDCl_3$) δ: 1.06(3H, s), 1.10(3H, s), 1.43(3H, t, J=6.9Hz), 1.48-1.58(3H, m), 1.87-2.05(2H, m), 2.38-2.41(1H, m), 3.17 (1H, dd, J=5.6, 14.2Hz), 3.23(1H, q, J=5.6Hz), 3.32(1H, d, J=2.0Hz), 3.73(1H, dd, J=7.6, 14.2Hz), 4.06(2H, q, J=6.9Hz), 4.79(1H, brs), 6.25-6.29(1H, m), 6.59(1H, d, J=8.3Hz), 6.90(2H, d, J=8.9Hz), 7.21(1H, dd, J=1.7, 8.3Hz), 7.31(1H, d, J=1.7Hz), 7.67(2H, d, J=8.9Hz). |
| 8 | 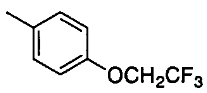 | ($CDCl_3$) δ: 1.07(3H, s), 1.11(3H, s), 1.52-1.74(4H, m), 1.84-1.98(2H, m), 2.37-2.45(1H, m), 3.16-3.26(2H, m), 3.32(1H, d, J=2.0Hz), 3.73(1H, dd, J=7.6, 14.3Hz), 4.16(2H, q, J=7.9Hz), 6.32(1H, t, J=6.6Hz), 6.60(1H, d, J=8.6Hz), 6.97(2H, d, J=8.9Hz), 7.21(1H, dd, J=1.7, 8.3Hz), 7.32(1H, s), 7.72(2H, d, J=8.6Hz). |
| 9 | 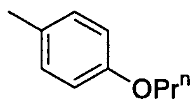 | ($CDCl_3$) δ: 1.04(3H, t, J=7.6Hz), 1.06(3H, s), 1.10(3H, s), 1.51-1.97(8H, m), 2.36-2.41(1H, m), 3.14-3.25(2H, m), 3.32 (1H, d, J=2.0Hz), 3.74(1H, dd, J=7.6, 14.2Hz), 3.95(2H, t, J=6.6Hz), 6.30(1H, t, J=6.6Hz), 6.62(1H, d, J=8.6Hz), 6.90(2H, d, J=8.6Hz), 7.22(1H, dd, J=1.7, 8.3Hz), 7.32(1H, s), 7.67(2H, d, J=8.9Hz). |
| 10 | 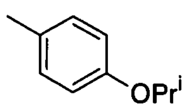 | ($CDCl_3$) δ: 1.06(3H, s), 1.09(3H, s), 1.34(6H, d, J=5.9Hz), 1.48-1.70(4H, m), 1.73-1.95(2H, m), 2.35-2.41(1H, m), 3.13-3.25(2H, m), 3.32(1H, d, J=2.3Hz), 3.74(1H, dd, J=7.6, 14.2Hz), 4.60(1H, m), 6.29(1H, t, J=6.6Hz), 6.61(1H, d, J=8.2Hz), 6.88(2H, d, J=8.9Hz), 7.21(1H, dd, J=1.7, 8.9Hz), 7.31(1H, s), 7.66(2H, d, J=8.9Hz). |
| 11 | 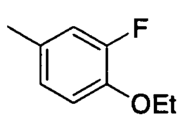 | ($CDCl_3$) δ: 1.07(3H, s), 1.10(3H, s), 1.47(3H, t, J=7.6Hz), 1.52-1.74(4H, m), 1.84-1.97(2H, m), 2.37-2.42(1H, m), 3.18-3.26(2H, m), 3.32(1H, d, J=2.3Hz), 3.71(1H, dd, J=7.3, 14.5Hz), 4.14(2H, q, J=6.9Hz), 6.36(1H, t, J=6.6Hz), 6.62(1H, d, J=8.6Hz), 6.95(1H, t, J=8.6Hz), 7.22(1H, dd, J=1.3, 8.2Hz), 7.32(1H, s), 7.45-7.50(2H, m). |
| 12 | 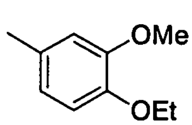 | ($CDCl_3$) δ: 1.06(3H, s), 1.10(3H, s), 1.48(3H, t, J=6.9Hz), 1.55-1.71(4H, m), 1.74-1.93(2H, m), 2.41(1H, d, J=7.3Hz), 3.17-3.25(2H, m), 3.33(1H, s), 3.71(1H, dd, J=7.3, 10.3Hz), 3.90(3H, s), 4.13(2H, q, J=6.9Hz), 4.75(1H, brs), 6.30(1H, brs), 6.60(1H, d, J=8.3Hz), 6.84(1H, d, J=8.3Hz), 7.20(2H, t, J=8.3Hz), 7.34(2H, d, J=7.3Hz). |

| | | |
|---|---|---|
| 13 | 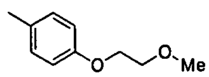 | ($CDCl_3$) δ: 1.05(3H, s), 1.09(3H, s), 1.43-1.73(4H, m), 1.83-1.92(2H, m), 2.33-2.43(1H, m), 3.18(1H, dd, J=5.9, 14.5Hz), 3.33(1H, d, J=2.0Hz), 3.45(3H, s), 3.61-3.80(3H, m), 4.11-4.20(2H, m), 4.48(1H, brs), 6.38(1H, brs), 6.59(1H, d, J=8.3Hz), 6.94(2H, d, J=8.9Hz), 7.20(1H, dd, J=2.0, 8.3Hz), 7.30(1H, s), 7.68(2H, d, J=8.9Hz), 8.03(1H, dd, J=2.0, 8.9Hz). |
| 14 | 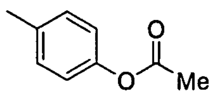 | ($CDCl_3$) δ: 1.07(3H, s), 1.10(3H, s), 1.52-1.74(4H, m), 1.88-1.96(2H, m), 2.32(3H, s), 2.37-2.42(1H, m), 3.20(2H, dd, J=5.6, 14.4Hz), 3.32(1H, d, J=2.0Hz), 3.75(1H, dd, J=7.3, 14.2Hz), 6.34(1H, t, J=6.6Hz), 6.61(1H, d, J=8.2Hz), 7.16(2H, d, J=8.6Hz), 7.22(1H, dd, J=1.7, 8.4Hz), 7.33(1H, s), 7.74(2H, d, J=8.6Hz). |
| 15 | 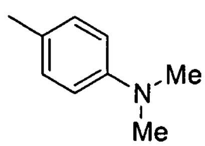 | ($CDCl_3$) δ: 1.04(3H, s), 1.09(3H, s), 1.42-1.72(4H, m), 1.87-1.99(2H, m), 2.35-2.39(1H, m), 3.01(6H, s), 3.14(2H, dd, J=5.6, 14.4Hz), 3.22-3.26(1H, m), 3.32(1H, d, J=1.7Hz), 3.75(1H, dd, J=7.6, 14.2Hz), 4.90(1H, brs), 6.22(1H, t, J=6.6Hz), 6.60(1H, d, J=8.6Hz), 6.65(2H, d, J=8.9Hz), 7.20(1H, dd, J=1.7, 8.4Hz), 7.30(1H, s), 7.62(2H, d, J=8.9Hz). |
| 16 | 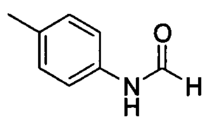 | ($CDCl_3$) δ: 1.07(3H, s), 1.10(3H, s), 1.51-1.74(4H, m), 1.83-1.99(2H, m), 2.36-2.43(1H, m), 3.15-3.29(1H, m), 3.33(1H, d, J=2.0Hz), 3.74(1H, dd, J=7.6, 14.3Hz), 6.53-6.58(1H, m), 6.62(1H, d, J=8.2Hz), 7.15(1H, dd, J=8.6, 14.6Hz), 7.31(1H, s), 7.56-7.75(4H, m), 8.02(1H, s). |
| 17 | 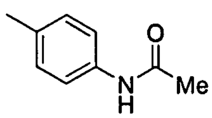 | (DMSO-$d_6$) δ: 0.97(3H, s), 0.99(3H, s), 1.37-1.44(2H, m), 1.58-1.70(2H, m), 1.87-1.99(2H, m), 2.37(1H, brs), 2.50(3H, t, J=1.7Hz), 3.23(2H, dd, J=6.3, 13.5Hz), 3.39(1H, s), 3.45(1H, dd, J=6.3, 13.5Hz), 6.15(1H, s), 6.81(1H, d, J=8.6Hz), 7.23(1H, dd, J=1.7, 8.4Hz), 7.35(1H, s), 7.63(2H, d, J=8.6Hz), 7.78(2H, d, J=8.6Hz), 8.21(1H, t, J=6.3Hz), 10.14(1H, s). |
| 18 | 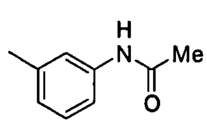 | ($CDCl_3$) δ: 1.06(3H, s), 1.11(3H, s), 1.51-1.73(4H, m), 1.88-1.96(2H, m), 2.20(3H, s), 2.40(1H, d, J=8.3Hz), 3.15-3.26(2H, m), 3.32(1H, d, J=2.3Hz), 3.74(1H, dd, J=7.3, 14.4Hz), 4.76(1H, brs), 6.50(1H, brs), 6.62(1H, d, J=8.2Hz), 7.21(1H, dd, J=2.0, 8.3Hz), 7.32-7.47(4H, m), 7.60(1H, d, J=7.9Hz), 7.96(1H, s). |

| | | |
|---|---|---|
| 19 | 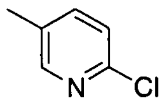 | ($CDCl_3$) δ: 1.08(3H, s), 1.12(3H, s), 1.52-1.75(4H, m), 1.89-2.00(2H, m), 2.38-2.44(1H, m), 3.25-3.34(3H, m), 3.71(1H, dd, J=6.9, 14.2Hz), 6.46(1H, brs), 6.59(1H, d, J=8.3Hz), 7.22(1H, dd, J=2.0, 8.3Hz), 7.33(1H, s), 7.42(1H, d, J=8.3Hz), 8.04(1H, dd, J=2.3, 8.3Hz), 8.70(1H, d, J=2.6Hz). |
| 20 | 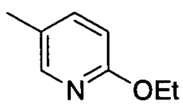 | (DMSO-$d_6$) δ: 0.95(3H, s), 0.98(3H, s), 1.23(3H, t, J=6.9Hz), 1.36-1.43(2H, m), 1.60-1.87(2H, m), 1.87(2H, brs), 2.33(1H, brs), 3.17-3.25(2H, m), 3.34-3.41(2H, m), 3.95(2H, q, J=6.9Hz), 6.15(1H, s), 6.38(1H, d, J=9.6Hz), 6.81(1H, d, J=8.3Hz), 7.22(1H, dd, J=1.3, 8.6Hz), 7.36(1H, s), 7.86(1H, dd, J=2.3, 9.4Hz), 8.06(1H, t, J=6.3Hz), 8.33(1H, d, J=2.3Hz). |
| 21 | 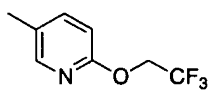 | ($CDCl_3$) δ: 1.10(3H, s), 1.15(3H, s), 1.47-1.73(4H, m), 1.84-2.02(2H, m), 2.39-2.42(1H, m), 3.19-3.27(2H, m), 3.33(1H, d, J=2.0Hz), 3.64(1H, dd, J=6.6, 14.4Hz), 4.50-4.74(3H, m), 6.32(1H, t, J=6.3Hz), 6.57(1H, d, J=8.3Hz), 6.60(1H, d, J=9.2Hz), 7.19(1H, dd, J=2.7, 8.3Hz), 7.31(1H, s), 7.58(1H, dd, J=2.6, 9.7Hz), 8.04(1H, s). |
| 22 | 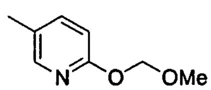 | ($CDCl_3$) δ: 1.05(3H, s), 1.10(3H, s), 1.53-1.69(4H, m), 1.73-1.99(2H, m), 2.36-2.42(1H, m), 3.15-3.39(3H, m), 3.40(3H, s), 3.68(1H, dd, J=6.9, 14.2Hz), 5.32(2H, s), 6.36(1H, t, J=6.3Hz), 6.57(2H, dd, J=9.6, 10.6Hz), 7.20(1H, dd, J=2.0, 8.4 Hz), 7.32(1H, s), 7.60(1H, dd, J=2.6, 9.6Hz), 8.10(1H, d, J=2.6Hz). |
| 23 | 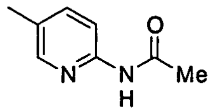 | (DMSO-$d_6$) δ: 0.98(3H, s), 1.00(3H, s), 1.15-1.25(2H, m), 1.46-1.67(2H, m), 1.83-1.99(2H, m), 2.11(3H, s), 2.35-2.38(1H, m), 3.16-3.49(4H, m),6.17(1H, s), 6.82(1H, d, J=8.3Hz), 7.23(1H, dd, J=2.0, 8.4Hz), 7.36(1H, s), 8.08-8.20(2H, m), 8.39(1H, t, J=6.3Hz), 8.75(1H, d, J=1.6Hz), 10.8(1H, s). |
| 24 | 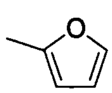 | ($CDCl_3$) δ: 1.06(3H, s), 1.10(3H, s), 1.52-1.73(4H, m), 1.19(2H, brs), 2.40(1H, d, J=7.6Hz), 3.13-3.25(2H, m), 3.34(1H, d, J=1.7Hz), 3.68(1H, dd, J=7.6, 14.2Hz), 4.67(1H, brs), 6.51(1H, dd, J=1.7, 3.6Hz), 6.58(2H, d, J=8.6Hz), 7.10(1H, d, J=3.6Hz), 7.21(1H, d, J=8.6Hz), 7.32(1H, s), 7.44(1H, s). |

| | | |
|---|---|---|
| 25 | 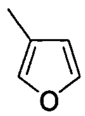 | ($CDCl_3$) δ: 1.05(3H, s), 1.09(3H, s), 1.47-1.73(4H, m), 1.85-1.98(2H, m), 2.36-2.41(1H, m), 3.16(1H, dd, J=6.3, 14.2Hz), 3.25(1H, t, J=5.6Hz), 3.33(1H, d, J=2.0Hz), 3.67(1H, dd, J=7.6, 14.4Hz), 6.11(1H, brs), 6.60(2H, d, J=8.6Hz), 7.21(1H, dd, J=2.0, 8.4 Hz), 7.32(1H, s), 7.44(1H, t, J=1.7Hz), 7.92(1H, dd, J=0.7, 1.5Hz). |
| 26 | 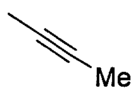 | ($CDCl_3$) δ: 1.01(3H, s), 1.04(3H, s), 1.55-1.69(5H, m), 1.89-1.98(2H, m), 1.94(3H, s), 2.38(1H, brs), 3.02(1H, dd, J=5.9, 14.2Hz), 3.28(1H, t, J=5.9Hz), 3.29(1H, d, J=2.3Hz), 3.55(1H, dd, J=7.6, 14.2Hz), 4.51(1H, brs), 5.88(1H, brs), 7.20(1H, dd, J=1.3, 8.3Hz), 7.33(1H, d, J=1.3Hz). |
| 27 | 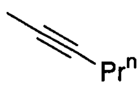 | ($CDCl_3$) δ: 0.96-1.04(9H, m), 1.43-1.96(6H, m), 1.88-2.19(2H, m), 2.25(1H, d, J=7.3Hz), 2.28(1H, d, J=6.9Hz), 2.31-2.38(1H, m), 3.03(1H, dd, J=5.9, 14.2Hz), 3.29-3.30(2H, m), 3.55(1H, dd, J=7.9, 14.4Hz), 4.54(1H, brs), 5.91(1H, brs), 6.56(1H, d, J=8.6Hz), 7.21(1H, dd, J=2.0, 8.3Hz), 7.33(1H, s). |
| 28 | 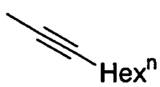 | ($CDCl_3$) δ: 0.88(3H, t, J=6.3Hz), 1.01(3H, s), 1.04(3H, s), 1.06-1.69(12H, m), 1.88-2.01(2H, m), 2.28(1H, t, J=6.9Hz), 2.34-2.40(1H, m), 3.03(1H, dd, J=6.3, 14.4Hz), 3.26-3.60(2H, m), 3.54(1H, dd, J=7.6, 14.4Hz), 4.55(1H, brs), 5.93(1H, brs), 6.56(1H, d, J=8.6Hz), 7.20(1H, dd, J=2.0, 8.4Hz), 7.33(1H, s). |
| 29 | 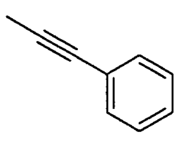 | ($CDCl_3$) δ: 1.03(3H, s), 1.07(3H, s), 1.50-1.65(4H, m), 1.84-1.86(2H, m), 2.37(1H, brs), 3.11(1H, dd, J=5.9, 14.2Hz), 3.20(1H, brs), 3.35(1H, s), 3.61(1H, dd, J=7.6, 14.2Hz), 4.71(1H, brs), 6.58(1H, d, J=8.6Hz), 6.73(1H, brs), 7.15(1H, d, J=8.3Hz), 7.28-7.39(4H, m), 7.47(2H, d, J=5.3Hz). |

[Example 30] Production of (3a*R**,4*S**,9b*S**)-1-[2-(8-cyano-2,3,3a,4,5,9b-hexahydro-1*H*-cyclopenta[*c*]quinolin-4-yl)-2-methylpropyl]-3-(3,4-dichlorophenyl)urea

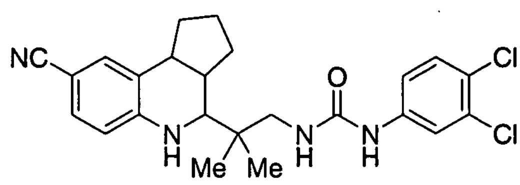

The compound (50.0 mg) obtained in (3) of Example 1 was dissolved in 2 mL of N,N-dimethylformamide, and 52 mg of 3,4-dichlorophenyl isocyanate was added. After the mixture was stirred for 1 hour at room temperature, water and ethyl acetate were added. The ethyl acetate layer was washed with water, and then dried over anhydrous magnesium sulfate. Then, the solvent was distilled off under reduced pressure. The residue was purified by silica gel column chromatography (elution solvent: hexane:ethyl acetate = 4:1 - 1:1) to obtain 72 mg of the captioned compound. Its physical properties are shown below.
$^1$H-NMR ($CDCl_3$) δ: 1.00 (3H, s), 1.13 (3H, s), 1.43-1.67 (6H, m), 2.38 (1H, d, J = 7.6 Hz), 3.11-3.23 (2H, m), 3.37 (1H, dd, J = 6.3, 13.9 Hz), 3.38 (1H, s), 4.56 (1H, s), 5.52 (1H, t, J = 6.3 Hz), 6.51 (1H, d, J = 8.6 Hz), 7.06 (1H, dd, J = 2.0, 8.4 Hz), 7.14 (1H, d, J = 2.3 Hz), 7.17 (1H, d, J = 2.3 Hz), 7.25 (1H, s), 7.29 (1H, d, J = 2.0 Hz), 7.52 (1H, d, J = 2.3 Hz).
Compounds shown in Examples 31 to 33 were produced in the same manner as in Example 30. The physical properties of the resulting compounds are shown in Table 2.

Table 2

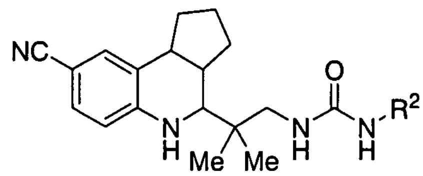

| Ex. No. | $R^2$ | $^1$H-NMR |
|---|---|---|
| 31 | 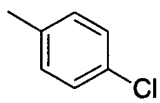 | ($CDCl_3$) δ: 0.98(3H, s), 1.00(3H, s), 1.43-2.00(6H, m), 2.35(1H, d, J=6.9Hz), 3.10(1H, dd, J=6.3, 14.4Hz), 3.20(1H, t, J=6.3Hz), 3.34-3.41(2H, m), 4.60(1H, s), 5.45(1H, t, J=6.3Hz), 6.49(1H, d, J=8.6Hz), 7.06(1H, d, J=8.6Hz), 7.17-7.26(5H, m). |
| 32 | 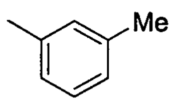 | ($CDCl_3$) δ: 0.93(3H, s), 0.95(3H, s), 1.37(2H, d, J=5.0Hz), 1.58-1.63(2H, m), 1.86(2H, brs), 2.23(1H, s), 2.31(1H, brs), 2.50(1H, d, J=1.7Hz), 3.08(1H, dd, J=6.3, 13.4Hz), 3.20-3.28(2H, m), 6.16(2H, d, J=7.3Hz), 6.69(1H, d, J=6.9Hz), 6.81(1H, d, J=8.6Hz), 7.04-7.14(2H, m), 7.24(2H, d, J=11.0Hz), 7.36(1H, s), 8.31(1H, s). |
| 33 | 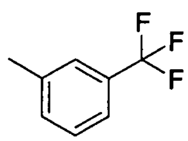 | ($CDCl_3$) δ: 1.02(3H, s), 1.04(3H, s), 1.44-1.51(2H, m), 1.66-1.78(2H, m), 1.94(2H, brs), 2.41(1H, d, J=6.3Hz), 2.58(1H, t, J=1.7Hz), 3.19(1H, dd, J=6.3, 13.5Hz), 3.30-3.50(2H, m), 6.23(1H, s), 6.41(1H, t, J=6.3Hz), 6.90(1H, d, J=8.2Hz), 7.29-7.32(2H, m), 7.44(1H, s), 7.51(2H, d, J=5.3Hz), 8.05(1H, s), 8.85(1H, s). |

Next, the usefulness of the compounds of the present invention will be described by the following test examples, which are by no means intended to limit the scope of the present invention.

[Test Example 1] Test for competitive binding to rat androgen receptors (rat AR)

Preparation of rat AR fraction: Prostates were harvested into ice-cooled ET buffer (10 mM Tris, 1 mM EDTA, 5 mM DTT, 10 mM sodium molybdate, pH 7.4) 3 days after orchiectomy in 11-week-old male SD rats. The prostate was finely cut, and ET buffer was added, whereafter the mixture was homogenized using a homogenizer. The homogenate was ultracentrifuged (100,000xg, 60 min, 4°C), and the supernatant was used as a rat AR fraction (hereinafter referred to as ARF).

Binding test: $^3$H-testosterone (hereinafter referred to as $^3$H-T) was diluted with ET buffer. Dihydrotestosterone (DHT) was prepared so as to have a concentration (final concentration 1 μM) 400 times the maximum concentration of $^3$H-T (2.5 nM). The $^3$H-T solution was added to a 1.5 ml tube containing DHT, no DHT, or various concentrations of a test compound. Further, 200 μg ARF was added to adjust the final volume to 100 μl. The mixture was incubated for 2 hours at 4°C, and then 300 μl of a 0.05% dextran T70-1.0% activated carbon solution was added. The mixture was further incubated for 15 minutes in ice to remove the free $^3$H-T by adsorption. After centrifugation (4°C, 2,500 rpm, 5 min), 275 μl of the supernatant was harvested into a liquid scintillation vial, and 2 ml of clear-sol was added. The mixture was stirred, allowed to stand, and measured for $^3$H radioactivity with a liquid scintillation counter.

Calculation of the relative binding inhibition rate: The binding inhibition rate (%) of the compound according to the present invention was calculated from the following equation, and the 50% inhibition concentration ($IC_{50}$) was calculated by the probit analysis of the concentration-binding inhibition curve.

Binding inhibition rate (%) = 100x[1-(a-c)/(b-c)] where

a: Radioactivity of a sample incorporating a compound of the present invention ($^3$H-T+compound)

b: Radioactivity of a sample containing no compound of the present invention ($^3$H-T alone: amount of total binding)

c: Radioactivity of a sample incorporating DHT ($^3$H-T+DHT: amount of nonspecific binding)

The relative binding inhibition rate (RBA: Relative Binding Affinity), with the binding inhibition rate of bicalutamide, which is an antiandrogen agent, being taken as 100, was obtained from the following equation (Endocrinology 138, 863-870, 1997):

$$RBA = 100 \times (IC_{50} \text{ of bicalutamide})/(IC_{50} \text{ of a compound of the present invention})$$

RBA's of the compounds of the present invention, calculated as above, are shown in Table 3.

Table 3

| Test Compound | RBA |
|---|---|
| Example 5 | 81 |
| Example 13 | 74 |
| Example 16 | 406 |
| Example 20 | 310 |
| Example 21 | 159 |
| Example 22 | 163 |
| Example 23 | 420 |
| Example 25 | 144 |
| Example 27 | 765 |
| Bicalutamide | 100 |

The compounds of the present invention were found to have very strong binding inhibition activity.

[Test Example 2] Effect on prostate, femoral bone mineral density, and levator ani muscle in orchiectomized (ORX) rats

Testes were removed from 12-week-old male SD rats. From the following day onward, the compound of Example 1 (30 mg/kg) or DHT (10 mg/kg) was subcutaneously administered once daily for 4 consecutive weeks on 5 days per week. The compound of the Example and the DHT were each dissolved in dimethyl sulfoxide, and then diluted 10-fold with olive oil. The resulting solution of each concentration was used in the test. In an ORX control group, dimethyl sulfoxide diluted 10-fold with olive oil was used for the test. The rats, which had not been orchiectomized, but had undergone laparotomy, followed by closure of the abdomen, were used as a sham control group. On the next day after final administration, the wet weights of the ventral prostate and the levator ani muscle were measured, and the bone mineral density of the right femur was measured by the DEXA method (dual energy X-ray absorptiometry), to evaluate the in vivo effect of the compound of Example 1. The results are shown in Table 4 and FIGs. 1 to 3.

Table 4

| Test compound | Prostate weight (mg/100 g body weight) | Femoral bone mineral density (mg/cm$^2$) | Levator ani muscle weight (mg/100 g body weight) |
|---|---|---|---|
| Sham control group | 94±17 | 140±6 | 53±4 |
| ORX control group | 8±1 | 128±4 | 35±4 |
| ORX+Example 1 30 mg/kg | 70±13** | 137±7** | 60±6** |
| ORX+DHT 10 mg/kg | 166±33** | 138±6** | 74±6** |

Mean±SD *$p$<0.05, **$p$<0.01 on Dunnett's t-test.

The compound of Example 1 showed the effect of significantly increasing the femoral bone mineral density and the weight of the levator ani muscle. At this time, the weight of the prostate was about 74% of that in the sham control group.

On the other hand, DHT administered in a dose of 10 mg/kg showed a significant increase in the femoral bone mineral density, and a significant increase in the weight of the levator ani muscle. However, the weight of the prostate increased to about 175% of that in the sham control group.

These results demonstrated that the compound of Example 1 did not show excessive action on the prostate, as observed with natural androgen, and that the compound of Example 1 showed strong growth action particularly on bone tissue and muscular tissue.

[Test Example 3] Effect on prostate, femoral bone mineral density, and levator ani muscle in orchiectomized (ORX) rats

Testes were removed from 12-week-old male SD rats. From the following day onward, the compound of Example 8 or 23 (30 mg/kg) or DHT (10 mg/kg) was subcutaneously administered once daily for 4 consecutive weeks on 5 days per week. The compounds of the Examples and the DHT were each dissolved in dimethyl sulfoxide, and then diluted 10-fold with olive oil. The resulting solution of each concentration was used in the test. In an ORX control group, dimethyl sulfoxide diluted 10-fold with olive oil was used for the test. The rats, which had not been orchiectomized, but had been subjected to laparotomy, followed by closure of the abdomen, were used as a sham control group. On the next day after final administration, the wet weights of the ventral prostate and the levator ani muscle were measured, and the bone mineral density of the right femur was measured by the DEXA method (dual energy X-ray absorptiometry), to evaluate the *in vivo* effects of the compounds of Examples 8 and 23.

The results are shown in Table 5 and FIGs. 4 to 6.

Table 5

| Test compound | Prostate weight (mg/100 g body weight) | Femoral bone mineral density (mg/cm$^2$) | Levator ani muscle weight (mg/100 g body weight) |
|---|---|---|---|
| Sham control group | 123±17 | 140±5 | 60±4 |
| ORX control group | 8±2 | 131±6 | 38±4 |
| ORX+Example 8 30 mg/kg | 62±10** | 136±3* | 58±6** |
| ORX+Example 23 30 mg/kg | 111±14** | 137±5* | 72±6** |
| ORX+DHT 10 mg/kg | 162±27** | 138±6** | 85±9** |

Mean±SD *$p$<0.05,
**$p$<0.01 on Dunnett's t-test.

The compound of Example 8 showed the effect of significantly increasing the femoral bone mineral density and the weight of the levator ani muscle. At this time, the weight of the prostate was about 50% of that in the sham control group. The compound of Example 23 also produced significant increases in the femoral bone mineral density and the weight of the levator ani muscle. At this time, the weight of the prostate was about 90% of that in the false operation control group.

On the other hand, DHT administered in a dose of 10 mg/kg showed a significant increase in the femoral bone mineral density, and a significant increase in the weight of the levator ani muscle. However, the weight of the prostate increased to about 130% of that in the sham control group.

These results demonstrated that the compounds of Examples 8 and 23 did not show excessive action on the prostate, as observed with natural androgen, and that these compounds showed strong growth action particularly on bone tissue and muscular tissue.

[Test Example 4] Effect of the oral administration on prostate, femoral bone mineral density, and levator ani muscle in orchiectomized (ORX) rats

Testes were removed from 12-week-old male SD rats. From the following day onward, the compound of Example 16 (30 mg/5 ml/kg) was orally administered twice daily for 4 consecutive weeks on 7 days per week. The test compound was suspended in a 0.5% methylcellulose solution as a solvent, and used in the test. In an ORX control group, a 0.5% methylcellulose solution (5 ml/kg) was administered. The rats, which had not been orchiectomized, but

had been subjected to laparotomy, followed by closure of the abdomen, were used as a sham control group. On the next day after final administration, the wet weights of the ventral prostate and the levator ani muscle were measured, and the bone mineral density of the right femur was measured by the DEXA method (dual energy X-ray absorptiometry), to evaluate the in vivo effect of the compound of Example 16. The results are shown in Table 6 and FIGs. 7 to 9.

Table 6

| Test compound | Prostate weight (mg/100 g body weight) | Femoral bone mineral density ($mg/cm^2$) | Levator ani muscle weight (mg/100 g body weight) |
|---|---|---|---|
| Sham control group | 111±14 | 140±4 | 55±5 |
| ORX control group | 8±4 | 132±6 | 34±4 |
| ORX+Example 16 30 mg/kg | 90±24** | 136±10* | 60±6** |

Mean±SD *$p$<0.05,
**$p$<0.01 on Dunnett's t-test.

The compound of Example 16 significantly increased the femoral bone mineral density and the weight of the levator ani muscle of the ORX rats, and restored these parameters to levels comparable to those in the sham control group. On the other hand, the weight of the prostate in the rats receiving the compound of Example 16 was about 90% of that in the sham control group.

These results demonstrated that the compound of Example 16, when administered orally, showed no excessive action on the prostate, and showed strong growth action particularly on bone tissue and muscular tissue.

The nonsteroidal AR agonists now under research and development are poorly absorbed in vivo when administered orally, and are thus applied as intravenous or

Preparation examples of the compound of the present invention will be shown below, but formulations of the compound are not restricted to them.

[Preparation Example 1] Tablets

In accordance with the following formulation, tablets containing 2 mg of an active ingredient per tablet were prepared:

| | |
|---|---|
| Compound of Example 1 | 2 mg |
| Starch | 48 mg |
| Lactose | 30 mg |
| Microcrystalline cellulose | 15 mg |
| Methylcellulose | 3 mg |
| Magnesium stearate | 2 mg |
| Total amount | 100 mg |

[Preparation Example 2] Capsules

In accordance with the following formulation, 100 mg of an ingredient mixture containing 2 mg of an active ingredient per capsule were encapsulated to prepare capsules:

| | |
|---|---|
| Compound of Example 1 | 2 mg |
| Starch | 38 mg |
| Lactose | 50 mg |
| Microcrystalline cellulose | 8 mg |
| Magnesium stearate | 2 mg |
| Total | 100 mg |

## INDUSTRIAL APPLICABILITY

The tetrahydroquinoline derivatives of the present invention, and pharmaceuticals containing them as active

ingredients can show an AR agonistic action without exerting excessive action on the prostate as observed with androgen steroid preparations. They can also show potent AR agonistic action particularly on bone tissue and skeletal muscle tissue. Hence, the compounds of the present invention can prevent or treat various diseases against which their AR agonistic action is expected to be effective. In the prevention or treatment of hypogonadism, they are applicable as pharmaceuticals with moderate action on the prostate and with fewer adverse effects. In the prevention or treatment of osteoporosis and wasting diseases, they can be expected to show potent action on target tissues, such as bone tissue and skeletal muscle tissue.

## Claims

1. A tetrahydroquinoline derivative represented by the following formula (I), or pharmacologically acceptable salts thereof:

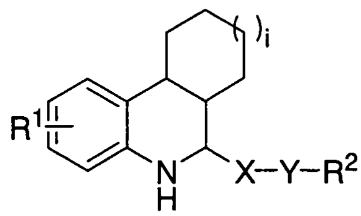

(I)

where $R^1$ represents a nitro group or a cyano group;

i represents 0 or 1;

X represents an alkylene group having 1 - 5 carbon atoms which may be substituted by a substituent selected from the group consisting of an alkyl group having 1 - 6 carbon atoms and a cycloalkyl group having 3 - 7 carbon atoms;

Y represents $-NR^3CO-$, $-NR^3SO_2-$, $-NR^3CONH-$ or $-NR^3CSNH-$ (where $R^3$ represents a hydrogen atom, an alkyl group having 1 - 6 carbon atoms, a cycloalkyl group having 3 - 7 carbon atoms, or an aralkyl group having 7 - 9 carbon atoms); and

$R^2$ represents a phenyl group which may be substituted by 1 - 3 independent $R^4$'s, or a heteroaryl group which may be substituted by 1 - 3 independent $R^{4'}$'s [where $R^4$ and $R^{4'}$ independently represent an alkyl group having 1 - 6 carbon atoms which may be substituted by a fluorine atom, a halogen atom, a nitro group, a cyano group, $-A-R^{5A}$ {where A is -CO-, $-CO_2-$, $-CONR^6-$, -O-, -OCO-, $-NR^6-$, $-NR^6CO-$, $-NR^6SO_2-$, $-NR^6CONH-$, $-NR^6CSNH-$ or $-NR^6COO-$ (where $R^6$ independently has the same meaning as the aforementioned $R^3$), and $R^{5A}$ represents a hydrogen atom, an alkyl group having 1 - 6 carbon atoms which may be substituted by a fluorine atom, or a cycloalkyl group having 3 - 7 carbon atoms}, or $-B-(CH_2)_n-R^{5B}$ {where B represents a single bond, -CO-, $-CO_2-$, $-CONR^{6'}-$, -O-, -OCO-, $-NR^{6'}-$, $-NR^{6'}CO-$, $-NR^{6'}SO_2-$, $-NR^{6'}CONH-$, $-NR^{6'}CSNH-$ or $-NR^{6'}COO-$ (where $R^{6'}$ independently has the same meaning as the aforementioned $R^3$), n represents an integer of 1 or 2, and $R^{5B}$ represents an alkyl group having 1 - 6 carbon atoms which may be substituted by a fluorine atom, a cycloalkyl group having 3 - 7 carbon atoms, a halogen atom, a hydroxyl group, a cyano group, an alkoxy group having 1 - 5 carbon atoms, or $-NR^{7'}R^{8'}$ (where $R^{7'}$ and $R^{8'}$ independently have the same meaning as the aforementioned $R^3$)}], or $-C\equiv C-R^9$ {where $R^9$ represents a hydrogen atom, an alkyl group having 1 - 6 carbon atoms which may be substituted by a fluorine atom, a cycloalkyl group having 3 - 7 carbon atoms, or an aryl group which may be substituted by $R^{10}$ (where $R^{10}$ represents an alkyl group having 1 - 6 carbon atoms which may be substituted by a fluorine atom, or a halogen atom)}.

2. The tetrahydroquinoline derivative or pharmacologically acceptable salts thereof according to claim 1, wherein i is 0, X is $-C(CH_3)_2-CH_2-$, Y is -NHCO- or -NHCONH-, and $R^2$ represents a phenyl group which may be substituted by 1 - 3 independent $R^4$'s, or a heteroaryl group which may be substituted by 1 - 3 independent $R^{4'}$'s [where $R^4$ and $R^{4'}$ independently represent an alkyl group having 1 - 6 carbon atoms which may be substituted by a fluorine atom, a halogen atom, $-A-R^{5A}$ {where A represents -CO-, -O-, -OCO-, $-NR^6-$, $-NR^6CO-$ or $-NR^6CONH-$ (where $R^6$ represents a hydrogen atom or a methyl group), and $R^{5A}$ represents a hydrogen atom, an alkyl group having 1 - 6 carbon atoms which may be substituted by a fluorine atom, or a cycloalkyl group having 3 - 7 carbon atoms}, or

$-B-(CH_2)_n-R^{5B}$ {where B represents -CO-, -O-, -OCO-, $-NR^{6'}-$, $-NR^{6'}CO-$ or $-NR^{6'}CONH-$ (where $R^{6'}$ represents a hydrogen atom or a methyl group), n represents an integer of 1 or 2, and $R^{5B}$ represents an alkyl group having 1 - 6 carbon atoms which may be substituted by a fluorine atom, a cycloalkyl group having 3 - 7 carbon atoms, or an alkoxy group having 1 - 5 carbon atoms}].

**3.** The tetrahydroquinoline derivative or pharmacologically acceptable salts thereof according to claim 1, wherein i is 0, X is $-C(CH_3)_2-CH_2-$, Y is -NHCO-, and $R^2$ is a phenyl group which may be substituted by 1 - 3 independent $R^4$'s, or a heteroaryl group which may be substituted by 1 - 3 independent $R^{4'}$'s [where $R^4$ and $R^{4'}$ independently represent an alkyl group having 1 - 6 carbon atoms which may be substituted by a fluorine atom, a halogen atom, $-A-R^{5A}$ {where A represents -CO-, -O-, -OCO-, -NH-, -NHCO- or -NHCONH-, and $R^{5A}$ represents a hydrogen atom, an alkyl group having 1 - 6 carbon atoms which may be substituted by a fluorine atom, or a cycloalkyl group having 3 - 7 carbon atoms}, or $-B-(CH_2)_n-R^{5B}$ {where B represents -CO-, -O-, -OCO-, -NH-, -NHCO- or -NHCONH-, n represents an integer of 1 or 2, and $R^{5B}$ represents an alkyl group having 1 - 6 carbon atoms which may be substituted by a fluorine atom, a cycloalkyl group having 3 - 7 carbon atoms, or an alkoxy group having 1 - 5 carbon atoms}].

**4.** The tetrahydroquinoline derivative or pharmacologically acceptable salts thereof according to claim 1, wherein i is 0, X is $-C(CH_3)_2-CH_2-$, Y is -NHCO-, and $R^2$ is a heteroaryl group which may be substituted by 1 - 3 independent $R^{4'}$'s {where $R^{4'}$ represents an alkyl group having 1 - 6 carbon atoms which may be substituted by a fluorine atom, a halogen atom, $-A-R^{5A}$ (where A represents -O- or -NHCO-, and $R^{5A}$ represents a hydrogen atom, or an alkyl group having 1 - 6 carbon atoms which may be substituted by a fluorine atom), or $-B-CH_2-R^{5B}$ (where B represents -O- or -NHCO-, and $R^{5B}$ represents an alkyl group having 1 - 6 carbon atoms which may be substituted by a fluorine atom, or an alkoxy group having 1 - 5 carbon atoms)}.

**5.** The tetrahydroquinoline derivative or pharmacologically acceptable salts thereof according to claim 3, wherein $R^2$ is a phenyl group having substituent $R^4$ at 4-position, or a 6-membered heteroaryl group having substituent $R^{4'}$ at 4-position [where $R^4$ and $R^{4'}$ independently represent a halogen atom, $-O-R^{5A}$, or $-NHCO-R^{5A}$ (where $R^{5A}$ represents a hydrogen atom, or an alkyl group having 1 - 6 carbon atoms which may be substituted by a fluorine atom)].

**6.** The tetrahydroquinoline derivative or pharmacologically acceptable salts thereof according to claim 3, wherein $R^2$ is a phenyl group having substituent $R^4$ at 4-position, or a 6-membered heteroaryl group having substituent $R^{4'}$ at 4-position [where $R^4$ and $R^{4'}$ independently represent $-NHCO-R^{5A}$ (where $R^{5A}$ represents a hydrogen atom, or an alkyl group having 1 - 6 carbon atoms which may be substituted by a fluorine atom)].

**7.** The tetrahydroquinoline derivative or pharmacologically acceptable salts thereof according to claim 1, wherein i is 0, X is $-C(CH_3)_2-CH_2-$, Y is -NHCO-, and $R^2$ is $-C\equiv C-R^9$ {where $R^9$ represents an alkyl group having 1 - 6 carbon atoms which may be substituted by a fluorine atom, or an aryl group which may be substituted by $R^{10}$ (where $R^{10}$ represents an alkyl group having 1 - 6 carbon atoms which may be substituted by a fluorine atom, or a halogen atom)}.

**8.** The tetrahydroquinoline derivative or pharmacologically acceptable salts thereof according to claim 7, wherein $R^9$ represents an alkyl group having 1 - 6 carbon atoms which may be substituted by a fluorine atom, or a phenyl group.

**9.** A pharmaceutical comprising the tetrahydroquinoline derivative or pharmacologically acceptable salts thereof according to any one of claims 1 to 8 as an active ingredient.

**10.** The pharmaceutical according to claim 9 which is an androgen receptor agonist.

**11.** The pharmaceutical according to claim 10 which can be used in preventing or treating osteoporosis or wasting disease.

**12.** The pharmaceutical according to claim 10 which can be used in preventing or treating a disease selected from the group consisting of male hypogonadism, male sexual dysfunction, abnormal sex differentiation, male delayed puberty, carcinoma of female genitalia, breast cancer, mastopathy, endometriosis and female sexual dysfunction.

**13.** The pharmaceutical according to claim 10 which can be used in preventing or treating hematopoietic dysfunction and a disease related thereto.

**14.** A method for preventing or treating wasting disease or osteoporosis, comprising administering the tetrahydroquinoline derivative or pharmacologically acceptable salts thereof according to any one of claims 1 to 8, in an amount effective for prevention or treatment of such disease, to a mammal requiring such prevention or treatment.

**15.** A method for preventing or treating a disease selected from the group consisting of male hypogonadism, male sexual dysfunction, abnormal sex differentiation, male delayed puberty, carcinoma of female genitalia, breast cancer, mastopathy, endometriosis and female sexual dysfunction, said method comprising administering the tetrahydroquinoline derivative or pharmacologically acceptable salts thereof according to any one of claims 1 to 8, in an amount effective for prevention or treatment of such disease, to a mammal requiring such prevention or treatment.

**16.** A method for preventing or treating hematopoietic dysfunction and a disease related thereto, said method comprising administering the tetrahydroquinoline derivative or pharmacologically acceptable salts thereof according to any one of claims 1 to 8, in an amount effective for prevention or treatment of such disease, to a mammal requiring such prevention or treatment.

FIG. 1

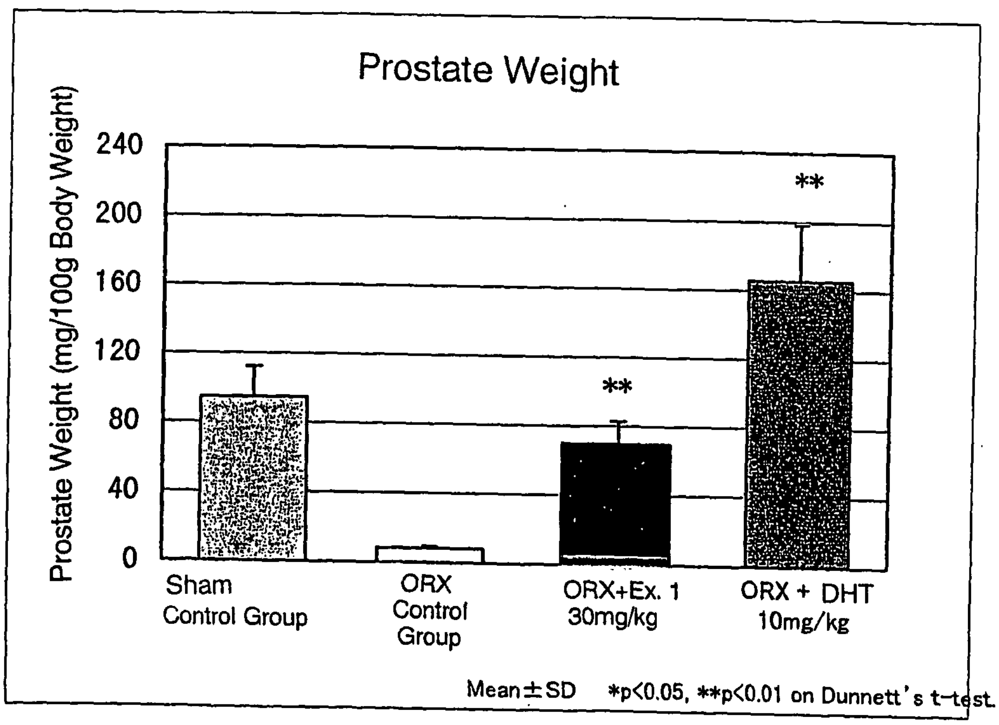
Prostate Weight
Prostate Weight (mg/100g Body Weight)
240
200
160
120
80
40
0
**
**
Sham Control Group
ORX Control Group
ORX+Ex. 1 30mg/kg
ORX + DHT 10mg/kg
Mean±SD *p<0.05, **p<0.01 on Dunnett's t-test

FIG. 2

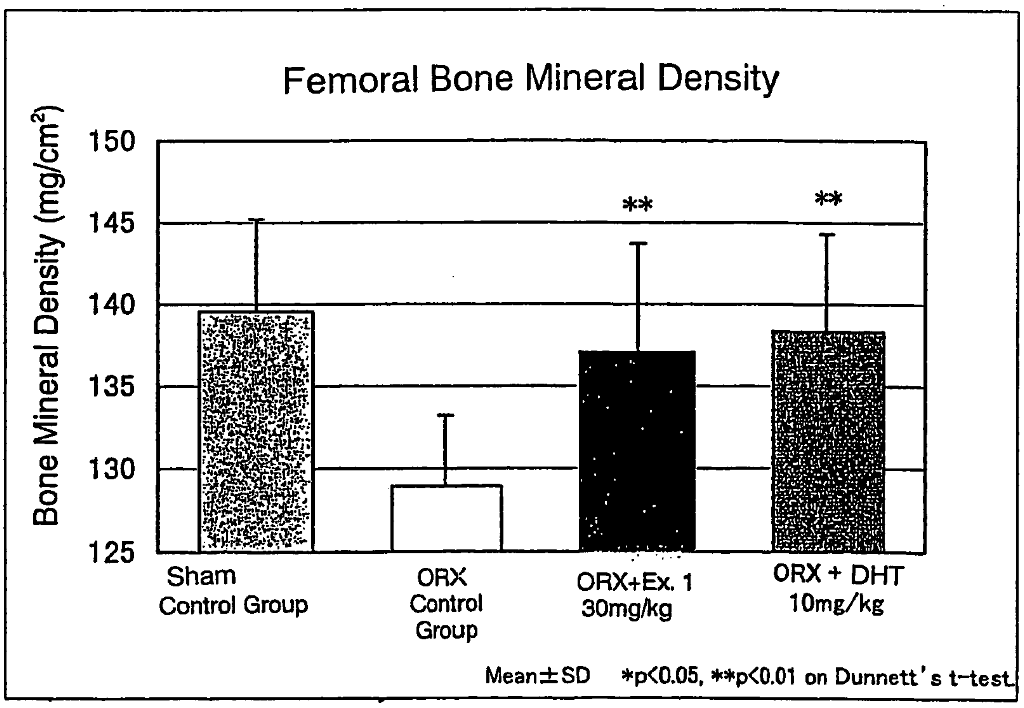
Femoral Bone Mineral Density
Bone Mineral Density (mg/cm²)
150
145
140
135
130
125
**
**
Sham Control Group
ORX Control Group
ORX+Ex. 1 30mg/kg
ORX + DHT 10mg/kg
Mean±SD *p<0.05, **p<0.01 on Dunnett's t-test.

FIG. 3

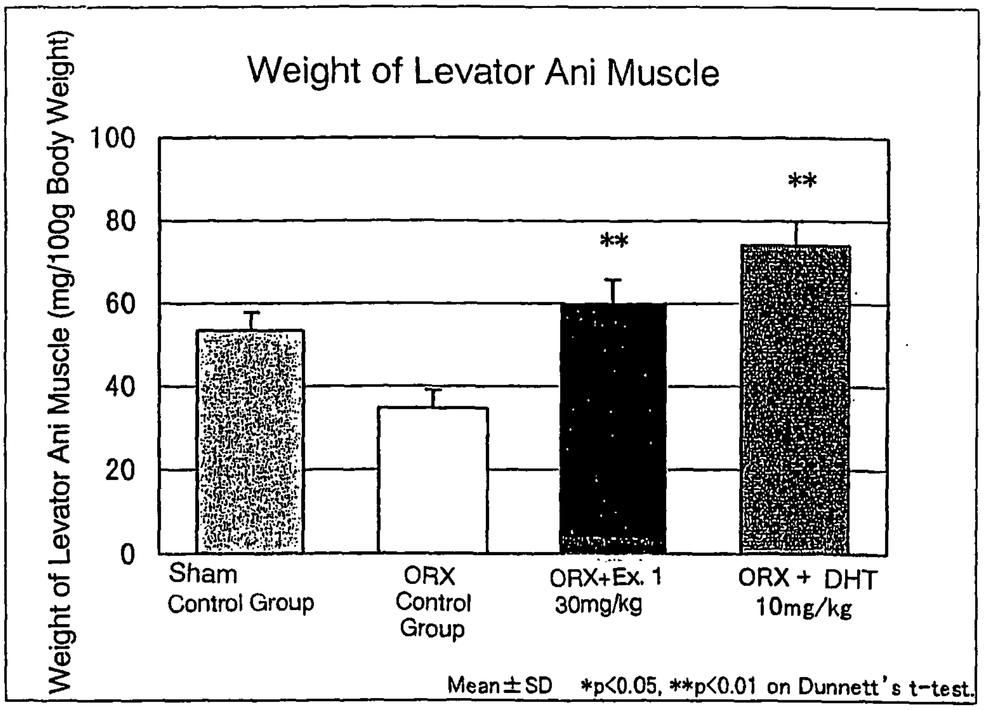
Weight of Levator Ani Muscle
Weight of Levator Ani Muscle (mg/100g Body Weight)
100
80
60
40
20
0
**
**
Sham
Control Group
ORX
Control
Group
ORX+Ex. 1
30mg/kg
ORX + DHT
10mg/kg
Mean±SD *p<0.05, **p<0.01 on Dunnett's t-test.

FIG. 4

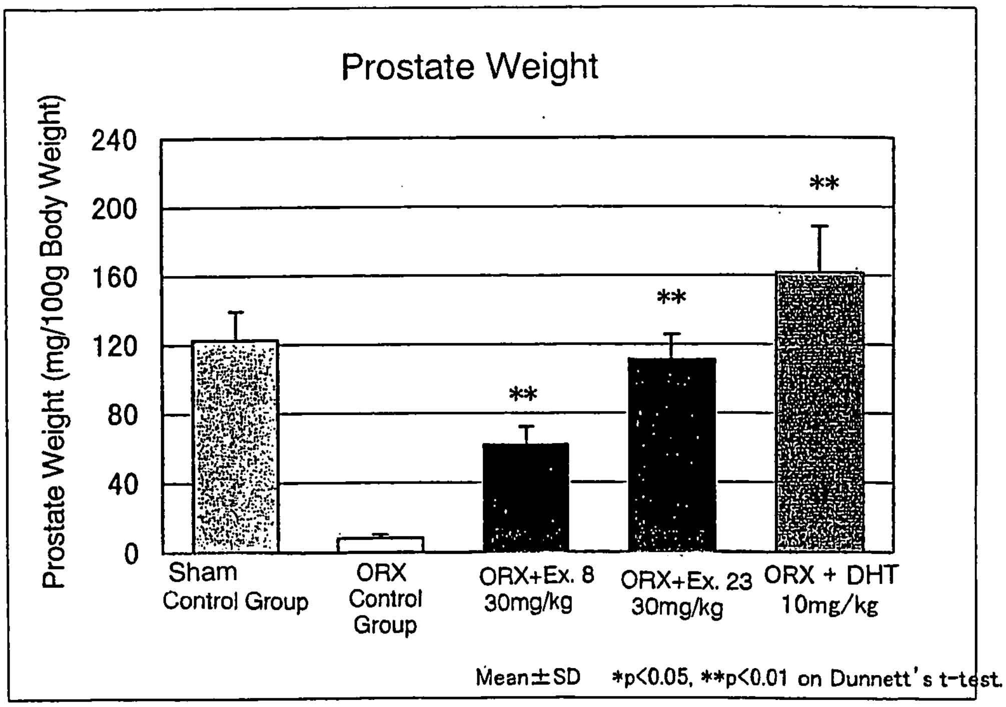
Prostate Weight
Prostate Weight (mg/100g Body Weight)
240
200
160
120
80
40
0
**
**
**
Sham
Control Group
ORX
Control
Group
ORX+Ex. 8
30mg/kg
ORX+Ex. 23
30mg/kg
ORX + DHT
10mg/kg
Mean±SD *p<0.05, **p<0.01 on Dunnett's t-test.

FIG. 5

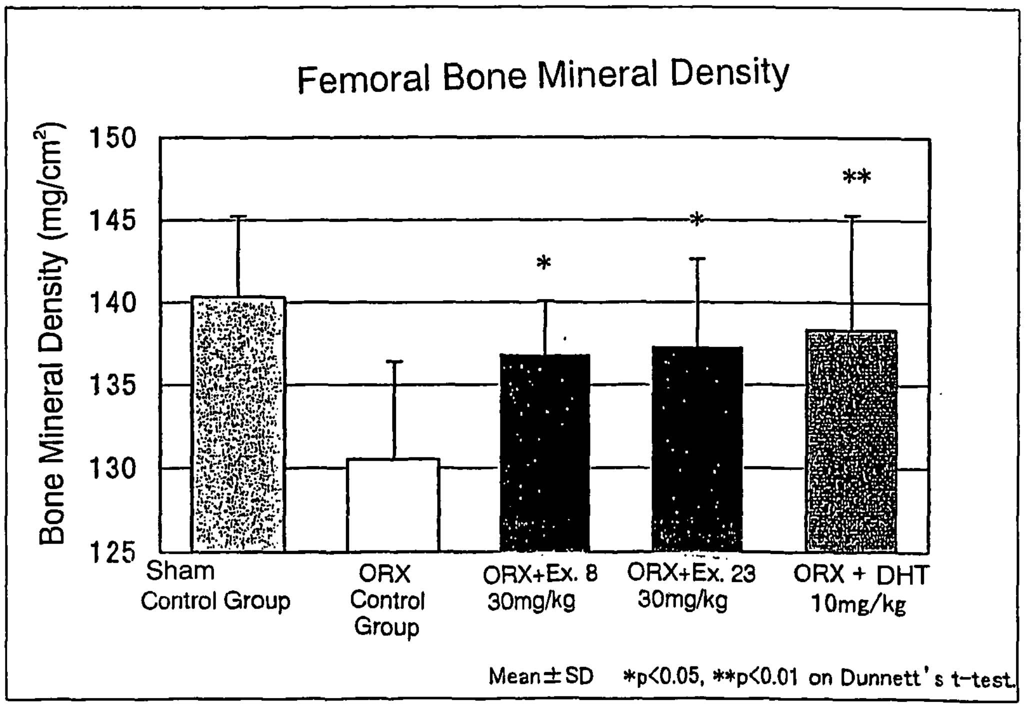
Femoral Bone Mineral Density
Bone Mineral Density (mg/cm²)
150
145
140
135
130
125
*
*
**
Sham Control Group
ORX Control Group
ORX+Ex. 8 30mg/kg
ORX+Ex. 23 30mg/kg
ORX + DHT 10mg/kg
Mean±SD *p<0.05, **p<0.01 on Dunnett's t-test

FIG. 6

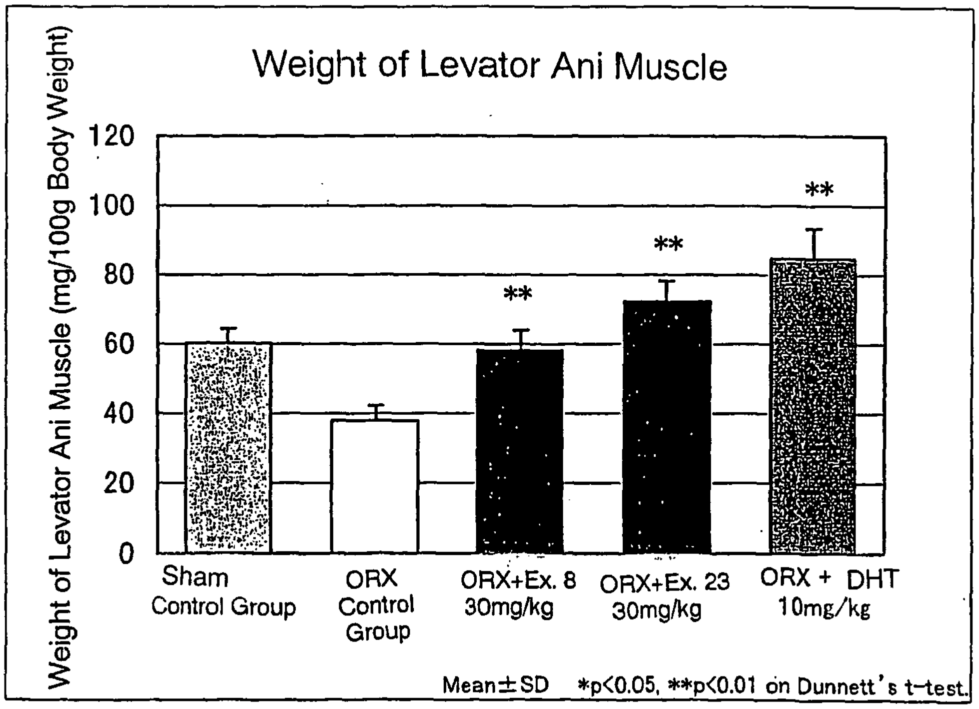
Weight of Levator Ani Muscle
Weight of Levator Ani Muscle (mg/100g Body Weight)
120
100
80
60
40
20
0
**
**
**
Sham Control Group
ORX Control Group
ORX+Ex. 8 30mg/kg
ORX+Ex. 23 30mg/kg
ORX + DHT 10mg/kg
Mean±SD *p<0.05, **p<0.01 on Dunnett's t-test.

FIG. 7

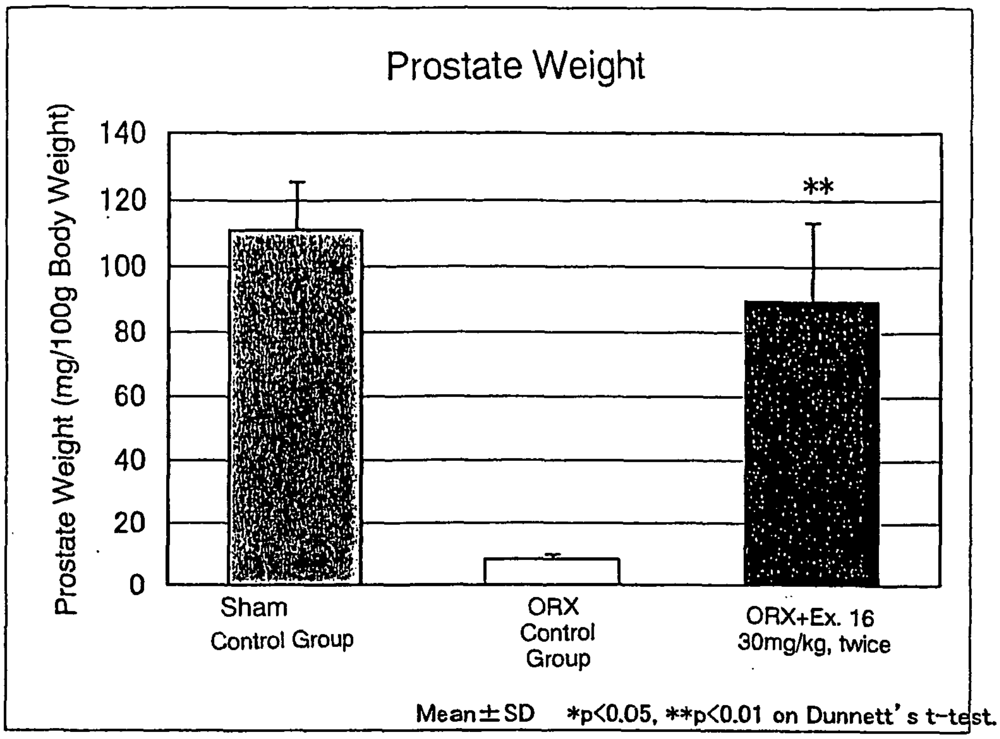
Prostate Weight
Prostate Weight (mg/100g Body Weight)
140
120
100
80
60
40
20
0
**
Sham
Control Group
ORX
Control
Group
ORX+Ex. 16
30mg/kg, twice
Mean±SD *p<0.05, **p<0.01 on Dunnett's t-test.

FIG. 8

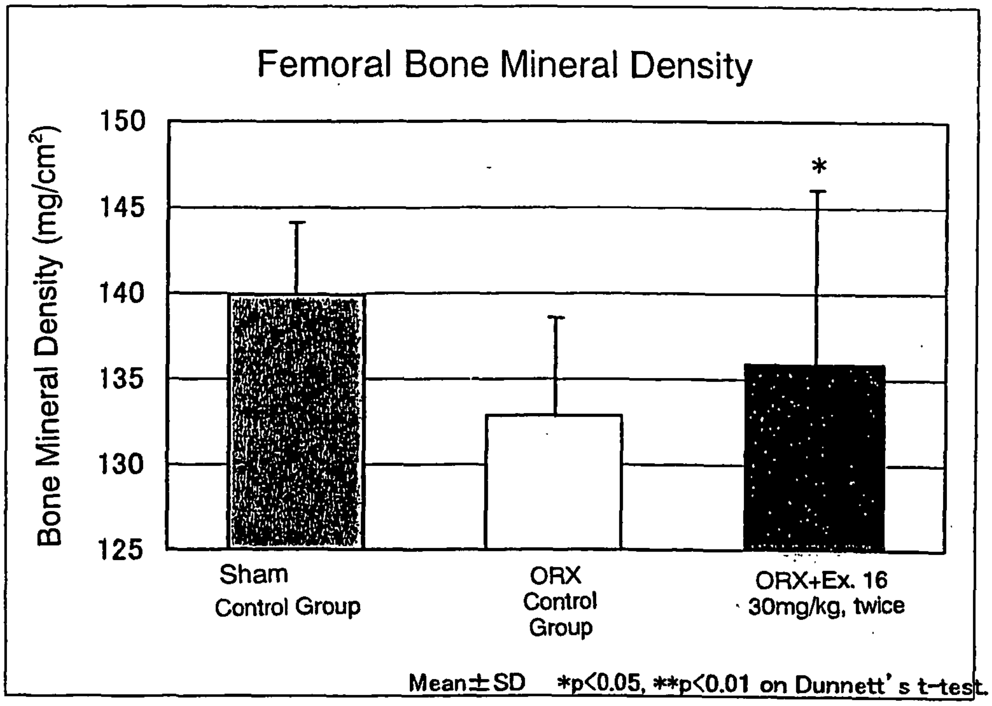
Femoral Bone Mineral Density
Bone Mineral Density (mg/cm²)
150
145
140
135
130
125
*
Sham
Control Group
ORX
Control
Group
ORX+Ex. 16
30mg/kg, twice
Mean±SD *p<0.05, **p<0.01 on Dunnett's t-test.

FIG. 9

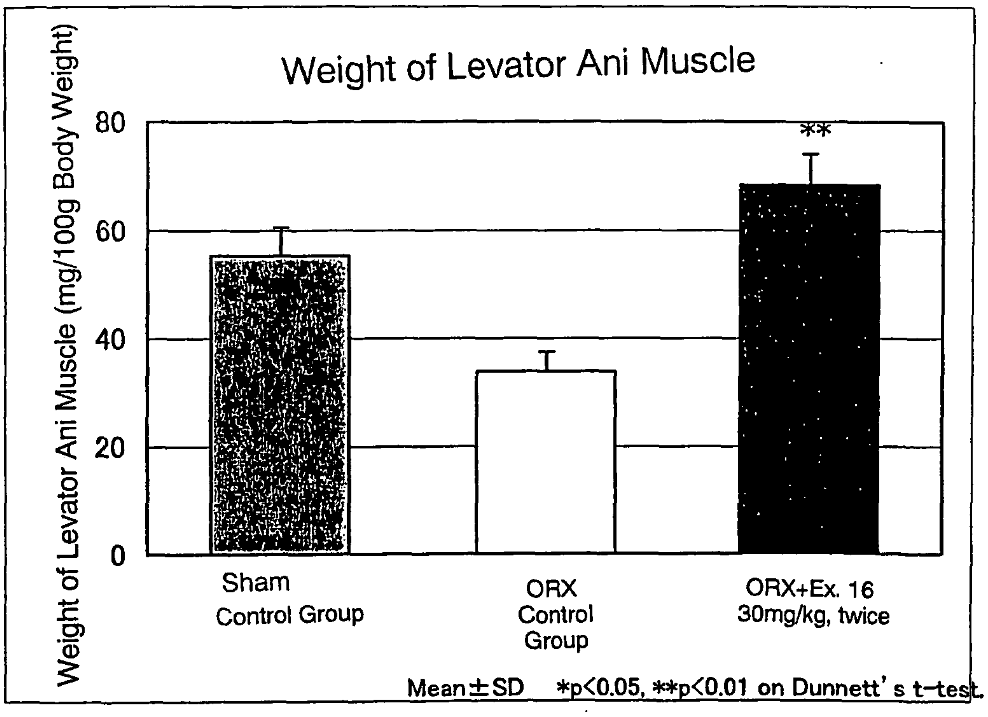
Weight of Levator Ani Muscle
Weight of Levator Ani Muscle (mg/100g Body Weight)
80
60
40
20
0
**
Sham
Control Group
ORX
Control
Group
ORX+Ex. 16
30mg/kg, twice
Mean±SD *p<0.05, **p<0.01 on Dunnett's t-test.

## INTERNATIONAL SEARCH REPORT

International application No.
PCT/JP03/09815

A. CLASSIFICATION OF SUBJECT MATTER
Int.Cl$^7$ C07D221/16, C07D401/12, C07D405/12, A61K31/473, A61P5/24, A61P7/00, A61P7/06, A61P15/00, A61P15/10, A61P19/10, A61P35/00, A61P43/00

According to International Patent Classification (IPC) or to both national classification and IPC

B. FIELDS SEARCHED

Minimum documentation searched (classification system followed by classification symbols)
Int.Cl$^7$ C07D221/16, C07D401/12, C07D405/12, A61K31/473, A61P5/24, A61P7/00, A61P7/06, A61P15/00, A61P15/10, A61P19/10, A61P35/00, A61P43/00

Documentation searched other than minimum documentation to the extent that such documents are included in the fields searched

Electronic data base consulted during the international search (name of data base and, where practicable, search terms used)
CAPLUS(STN), CAOLD(STN), REGISTRY(STN)

C. DOCUMENTS CONSIDERED TO BE RELEVANT

| Category* | Citation of document, with indication, where appropriate, of the relevant passages | Relevant to claim No. |
|---|---|---|
| X | EP 1221439 A1 (Kaken Pharmaceutical Co., Ltd.), 10 July, 2002 (10.07.02), Full text & WO 01/27086 A1 & AU 200075589 A & KR 2002056901 A & CN 1378535 A | 1-3,5,6,9-13 |
| A | WO 01/58875 A2 (GRUNENTHAL GMBH.), 16 August, 2001 (16.08.01), Full text & JP 2003-522758 A & AU 200126794 A & DE 10005302 A & EP 1254118 A2 & US 2003/0087926 A1 | 1-13 |

☐ Further documents are listed in the continuation of Box C. ☐ See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 23 October, 2003 (23.10.03) | 11 November, 2003 (11.11.03) |
| Name and mailing address of the ISA/ Japanese Patent Office | Authorized officer |
| Facsimile No. | Telephone No. |

Form PCT/ISA/210 (second sheet) (July 1998)

**INTERNATIONAL SEARCH REPORT**

International application No.
PCT/JP03/09815

**Box I Observations where certain claims were found unsearchable (Continuation of item 2 of first sheet)**

This international search report has not been established in respect of certain claims under Article 17(2)(a) for the following reasons:

1. [x] Claims Nos.: 14 to 16
because they relate to subject matter not required to be searched by this Authority, namely:
Claims 14 to 16 pertain to methods for treatment of the human body by therapy and thus relate to a subject matter which this International Searching Authority is not required to search.

2. [ ] Claims Nos.:
because they relate to parts of the international application that do not comply with the prescribed requirements to such an extent that no meaningful international search can be carried out, specifically:

3. [ ] Claims Nos.:
because they are dependent claims and are not drafted in accordance with the second and third sentences of Rule 6.4(a).

**Box II Observations where unity of invention is lacking (Continuation of item 3 of first sheet)**

This International Searching Authority found multiple inventions in this international application, as follows:

1. [ ] As all required additional search fees were timely paid by the applicant, this international search report covers all searchable claims.

2. [ ] As all searchable claims could be searched without effort justifying an additional fee, this Authority did not invite payment of any additional fee.

3. [ ] As only some of the required additional search fees were timely paid by the applicant, this international search report covers only those claims for which fees were paid, specifically claims Nos.:

4. [ ] No required additional search fees were timely paid by the applicant. Consequently, this international search report is restricted to the invention first mentioned in the claims; it is covered by claims Nos.:

**Remark on Protest** [ ] The additional search fees were accompanied by the applicant's protest.
[ ] No protest accompanied the payment of additional search fees.

Form PCT/ISA/210 (continuation of first sheet (1)) (July 1998)